# EUROPEAN PATENT APPLICATION

(11) **EP 2 016 954 A1**
(43) Date of publication of application: **21.01.2009**
(21) Application number: 07014089.2
(22) Date of filing: 18.07.2007
(51) Int. Cl.: A61K 39/44, A61K 39/395, A61K 31/4406, G01N 33/53, C07D 213/50, C07K 16/44, A61K 47/48

(54) **Antibodies against 4-(methylnitrosamino)-1-(3-pyridyl)-1-butanone and metabolites thereof**

(71) Applicant: Muller, Claude P., 1950 Luxembourg (LU); Centre de Recherche Publique de la Santé (CRP-Santé), 1445 Strassen (LU)
(72) Inventor: Muller, Claude P. Institute of Immunology, 1950 Luxembourg (LU); Ensch, Corinne, 66693 Mettlach-Orscholz (DE); Prodhomme, Emmanuel Institute of Immunology, 1950 Luxembourg (LU)
(74) Representative: Vossius & Partner

(57) **Abstract**

The present invention relates to compounds, methods for their preparation, conjugates obtainable by coupling the compounds to immunogenic carriers, pharmaceutical compositions comprising the conjugates, the use of the conjugates for treating and/or preventing disorders associated with tobacco specific nitrosamine exposure, methods for the preparation of the conjugates, antibodies which can be obtained using the conjugates, a method for the preparation of the antibodies and a method of detecting 4-(methylnitrosamino)-1-(3-pyridyl)-1-butanone (NNK), a metabolite or an adduct comprising a pyridyloxobutyl group derived from NNK, wherein the antibodies are employed. The present invention also relates to a method of detecting antibodies specific for NNK, non-detoxified metabolites or the above adducts thereof, wherein the conjugates are employed.

## Description

The present invention relates to compounds, methods for their preparation, conjugates obtainable by coupling the compounds to immunogenic carriers, pharmaceutical compositions comprising the conjugates, the use of the conjugates for treating and/or preventing disorders associated with tobacco specific nitrosamine exposure, methods for the preparation of the conjugates, antibodies which can be obtained using the conjugates, a method for the preparation of the antibodies and a method of detecting 4-(methylnitrosamino)-1-(3-pyridyl)-1-butanone (NNK), a metabolite or an adduct comprising a pyridyloxobutyl group derived from NNK, wherein the antibodies are employed. The present invention also relates to a method of detecting antibodies specific for NNK, non-detoxified metabolites or the above adducts thereof, wherein the conjugates are employed.

Overwhelming epidemiological and biological evidence exposes tobacco smoking as the main cause of the dramatic increase in lung cancer worldwide during the 20th century (Hecht S.S., Lancet Oncol 2002;3:461-469). In particular, TSNA (tobacco-specific nitrosamine) is favored as a major etiological factor in tobacco-induced lung cancer. TSNAs are present in unburned tobacco as well as mainstream and sidestream cigarette smoke with concentrations in current full-flavored cigarettes of about 130 ng per cigarette. The levels of TSNA, especially the nicotine-derived TSNA NNK (Hecht SS., J Natl Cancer Inst 1999:91:1194-1210), in tobacco are thousands of times higher than the amounts of other N-nitrosamines in consumer products that are regulated by government authorities. Exposure to NNK therefore represents an unacceptable risk to tobacco consumers, and possibly to non-smokers exposed for years to environmental tobacco smoke (ETS). The uptake of NNK by smokers has been clearly demonstrated and human lung and liver metabolically activates NNK.

NNK has a remarkable specificity, inducing predominantly adenocarcinomas in lungs of rats, mice and hamsters irrespective of the route of administration (Hecht SS et al., Carcinogenesis 1988;9:875-884). In addition, NNK and its major metabolite 4-(methylnitrosamino)-1-(3-pyridyl)-1-butanol (NNAL) are also the only known pancreatic carcinogens in cigarette smoke (Rivenson A. et al., Cancer Res 1988;48:6912-6917), and rats exposed to repetitive oral swabbing with a mixture of NNK and N'-nitrosonornicotine develop oral tumors (Hecht SS., et al., Cancer Res 1986;46:4162-4166). The total life-time dose of a smoker is remarkably close to the lowest total dose of NNK shown to induce tumors in rats (Hecht SS, Chem Res Toxicol 1998;11:559-603). NNK is generally considered a major carcinogen of the lung, oral cavity, and pancreas in active and passive smokers.

To exert its carcinogenic potential, NNK must be metabolically activated by α-hydroxylation at either the methyl or methylene carbons adjacent to the N-nitroso group, leading to the formation of electrophiles that are able to form DNA adducts (Hecht SS, Chem Res Toxicol 1998;11:559-603). For NNK, DNA-adducts in lung tissue of laboratory animals closely correlate with tumor formation (Belinsky S.A. et al., Cancer Res 1992;52:3164-3173; Belinsky S.A. et al., Cancer Res 1990;50:3772-3780; Peterson L.A. and Hecht S.S., Cancer Res 1991;51:5557-5564; Peterson L.A. et al, Cancer Res 2001;61:5757-5763). NNK-specific DNA adducts have also been found in smoker's lungs (Hecht, S.S., Crit Rev Toxicol 1996;26:163-181; Hecht S.S. et al., Environ Health Perspect 1993;99:57-63). Furthermore, methyl and pyridyloxobutyl DNA adducts have also been detected in human hemoglobin during NNK exposure. Mutations in the p53 and KRAS genes are supposedly caused by the activated metabolites of TSNA. Among the common lung cancer types, small cell carcinoma (SCLC) is found almost exclusively in smokers. This seems to be largely due to the induction of the proliferation of SCLC cells induced by tobacco-specific NNK upon binding to α7-nicotinic acetylcholine receptor selectively expressed in these cells (Schuller, H.M. and Orloff, M., Biochem Pharmacol 1998;55:1377-1384).

Collectively, these data provide strong evidence for a role of TSNA as cause of lung cancer in smokers.

In vivo, NNK is metabolized, either to detoxified species or activated metabolites responsible for its carcinogenic effect (Hecht, S. S. et al. (1994) Drug Metab Rev 26, 373-90). The metabolic pathways are similar in rodents and humans although with some quantitative differences (Prokopczyk, B. et al. (2001) Carcinogenesis 22, 107-14; Staretz, M. E. et al. (1997) Drug Metab Dispos 25, 154-62). NNK is first converted by carbonyl reduction to 4-(methylnitrosamino)-1-(3-pyridyl)-1-butanol (NNAL). NNK and NNAL are then further metabolized by α-hydroxylation of both carbons adjacent to the N-nitroso-group by cytochrome P450 enzymes (Jalas, J. R. et al. (2005) Chem Res Toxicol 18, 95-110). During this activation process several reactive electrophilic intermediates are generated that methylate and pyridyloxobutylate DNA (Hecht, S. S. (1999) Mutation Research/Fundamental and Molecular Mechanisms of Mutagenesis 424, 127-142). These DNA adducts may for instance inactivate p53 tumor-suppressor gene (Pfeifer, G. P. et al. (2002) Oncogene 21, 7435-51; Cloutier, J.-F. et al.. (1998) Chemico-Biological Interactions 110, 7-25) or activate k-ras protooncogene (Hecht, S. S. et al. (1993) Environ Health Perspect 99, 57-63; Peterson, L. A. et al. (2001) Cancer Res 61, 5757-63; Hashimoto, K. et al. (2004) Mutation Research/Genetic Toxicology and Environmental Mutagenesis 560, 119) both responsible for NNK carcinogenesis. Two other main metabolic pathways include pyridine N-oxidation to NNK-N-oxide and NNAL-N-oxide both of which are significantly less tumorigenic than their parent compounds and are therefore regarded as detoxification products. Glucuronidation of NNAL generates species that can be excreted via bile and urine, it is the main pathway for NNK detoxification in humans (Ren, Q. et al. (2000) Biochem J 349, 599-604).

Many chemopreventive strategies against chemical carcinogenesis have been explored. However, there are at present no agents to protect the lung against cancer in humans (Hecht S.S., Lancet Oncol 2002;3:461-469). The risk for cancer related to NNK exposure depends on the dose, the individual activation and detoxification capacity and the extent of DNA adduct formation. Many agents have been tested for their ability to inhibit NNK carcinogenicity by chemoprevention (Li, L. et al., J Biochem Mol Toxicol (2005) 19: 396-405; Lippman, S. M. and Spitz, M. R., J Clin Oncol (2001) 19, 74S-82S) and some, such as phenethyl isothiocyanate (PEITC), showed some benefit in preliminary studies (Hecht, S. S. Proc Soc Exp Biol Med(1997) 216, 181-91.). Further agents that have failed in clinical trials include N-acetylcysteine, 13-cis-retinoic acid, retinyl palmitate and β-carotene (Hecht S.S., Lancet Oncol 2002;3:461-469). An alternative approach that so far has received little attention relies on antibodies that would bind and sequester carcinogens, cf. Silbart L.K. et al., Vet Hum Toxicol 1997;39:37-43. Vaccination against nicotine has spurred a renewed interest in prophylactic strategies against low molecular weight compounds and several vaccines are currently under development (Cerny, E. H. et al., Onkologie (2002) 25, 406-11; Cerny, T., Recent Results Cancer Res (2005) 166, 167-75; Hall, W., Lancet (2002) 360, 1089-91; Maurer, P. et al., Eur J Immunol (2005) 35, 2031-40; Kantak, K. M., Drugs (2003) 63, 341-52). The aim of such prophylactic vaccines is to interrupt the drug-induced dependency of nicotine by preventing the molecule from reaching the central nervous system (Hieda, Y. et al., Int J Immunopharmacol (2000) 22, 809-19; Malin, D. H. et al., Pharmacol Biochem Behav (2001) 68, 87-92; Pentel, P. and Malin, D., Respiration (2002) 69, 193-7; Pentel, P. R. et al., Pharmacol Biochem Behav (2000) 65, 191-8).

Several attempts have been made in the past to generate antibodies against nicotine and its derivatives (Langone, J. J. et al., Methods in Enzymology (1982) 84, 628-650). For example, Langone J. J. et al., Biochemistry (1973) 12, 5025-5030 describe antibodies raised against nicotine and cotinine coupled to inter alia serum albumine for diagnostic purposes. The antibody described in EP-A-1 512 414 was raised in response to a nicotine hapten-carrier conjugate in order to provide a vaccine to prevent and treat nicotine addiction. Talbot, B. et al., Arch. Toxicol. (1990) 64, 360-364 describe polyclonal antibodies recognizing the pyridyloxobutyl moiety of NNK which are used for diagnostic purposes. US-A-2005/0043515 discloses an antibody produced using an NNK conjugate, wherein NNK was derivatized at the keto-group for coupling as a hapten to an immunogenic carrier. The obtained antibodies are said to be useful in assays for the detection of tobacco-specific nitrosamines. Due to the derivatization of the keto-group, however, a significant structural feature of NNK and NNAL, i.e. the keto-group and the hydroxyl-group, are not available during the raising of the antibody.

In summary, whereas the prior art has provided insight into the metabolic pathways of NNK and its derivatives, so far no promising means have been developed to allow for the prevention or treatment of diseases induced by NNK and/or metabolites thereof. Accordingly, one problem to be solved by the present invention is the provision of such means and methods.

A further problem to be solved by the present invention is the provision of novel NNK conjugates which facilitate the preparation of improved antibodies against NNK and/or metabolites thereof. In addition, the provision of means to detect NNK and/or metabolites thereof with higher sensitivity is another problem to be solved.

It has now surprisingly been found that solutions to the above problems can be achieved by providing the embodiments characterized in the claims.

In one aspect, the present invention relates to a compound of formula (I)

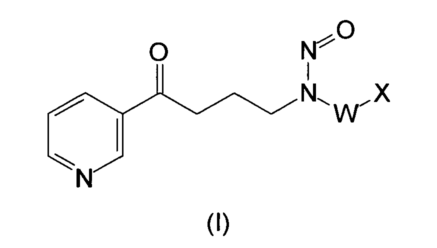

X is a functional group which allows coupling of the compound of formula (I) to an immunogenic carrier. For example, X can be selected from COOH, NH₂, SH, OH and halogen (i.e. F, Cl, Br and I, preferably Br and I). In a preferred embodiment, X is selected from COOH, NH₂ and SH. More preferably, X is NH₂.

W is C₁₋₁₂ alkylene, wherein one or more CH₂ are optionally replaced by O, provided that each O is separated from the N and the X, to which W is attached, and from the other O's, if present, by at least two CH₂. Preferably, W is C₁₋₁₂ alkylene or -(CH₂-CH₂-O)ₙ-CH₂-CH₂-, wherein n is an integer of 1 to 3. More preferably, W is C₁₋₁₂ alkylene, even more preferably C₂₋₆ alkylene and particularly preferably -CH₂-CH₂-.

In a further aspect, the present invention relates to a method for the preparation of the compound of formula (I), comprising:
nitrosating a compound of formula (II) 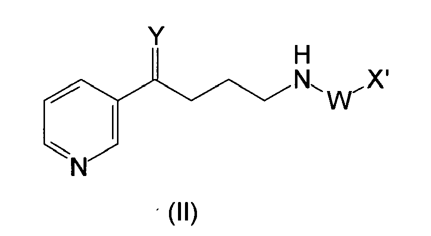 and removing the protecting groups to convert =Y into a carbonyl group and to convert X' into X.

X' is a protected functional group, wherein the functional group, after removal of the protecting group, allows coupling of the compound of formula (I) to an immunogenic carrier. Preferably, X' is COOR', NHR', SR', OR' or halogen, more preferably COOR', NHR' or SR', even more preferably NHR'. R' is a protecting group. When X' is halogen, X and X' are identical and no protecting group is removed from X' in the step of removing the protecting groups. The skilled artisan will readily be able to determine suitable groups that can be employed to protect the group X. In particular, he is familiar with suitable protecting groups for COOH, NH₂, SH and OH. Suitable protecting groups can, for example, be found in T. W. Greene and P. G. M. Wuts "Protective Groups in Organic Synthesis" 3rd Edition, June 1999, John Wiley & Sons Inc. Examples of protecting groups for COOH include ester groups, such as alkyl esters (e.g. methyl ester and t-butyl ester), methoxymethyl ester, methylthiomethyl ester, tetrahydropyranyl ester, benzyloxymethyl ester, acetol ester, phenyl ester and silyl esters (e.g. trimethylsilyl ester and 2-(trimethylsilyl)ethyl ester). Examples of protecting groups for NH₂ include carbamates, such as t-butyl carbamate, 9-fluorenylmethyl carbamate and allyl carbamate, and amides, such as formamide, acetamide, chloroacetamide, trichloroacetamide and trifluoroacetamide.

In a preferred embodiment, X' is NH-Boc, wherein Boc is butyloxycarbonyl. Examples of protecting groups for SH include thio ethers, such as S-alkyl thioether, S-benzyl thioether, S-diphenylmethyl thioether, S-t-butyl thioether, silyl thioether and their substituted forms. Examples of protecting groups for OH include ethers, such as methyl ether, ethyl ether, t-butyl ether, benzyl ether, silyl ether and their substituted forms.

=Y is a protected carbonyl group. The skilled artisan will readily be able to determine suitable groups that can be employed to protect the carbonyl group. The denotation =Y is intended to not only include protected carbonyl groups wherein the carbon atom of the protected carbonyl group forms a double bond with the protecting group, but also to include protecting groups wherein the carbon atom of the protected carbonyl group forms two single bonds to the protecting group. Suitable protecting groups can, for example, be found in T. W. Greene and P. G. M. Wuts "Protective Groups in Organic Synthesis" 3rd Edition, June 1999, John Wiley & Sons Inc. Examples of the protected carbonyl group include 1,3-dioxolane, dimethyl acetal, diisopropyl acetal, dibenzyl acetal, 1,3-dioxane, 5-trimethylsilyl-1,3-dioxane, 1,3-dithiane, 1,3-dithiolane and their substituted forms. In a particularly preferred embodiment, the protected carbonyl group is 1,3-dioxolan.

W is as defined for formula (I) above.

X is as defined for formula (I) above.

The step of nitrosating the compound of formula (II) can be carried out by using any of the protocols that are commonly available for nitrosating an amine group. For example, the compound of formula (II) can be nitrosated using an acid-catalyzed nitrosation, for example with NaNO₂ and catalytic amounts of hydrochloric acid under biphasic conditions. Alternative nitrosating methods include the use of nitrous acid, Fremy's salt, bis(triphenyl-phosphine)nitrogen nitrite, N-haloamides, oxyhyponitrite or dinitrogen tetroxide.

Suitable protocols to remove the protecting groups to convert =Y into a carbonyl group and to convert X' into X will, of course, depend on the specific protecting groups employed. Such protocols are known to the skilled artisan and are, for example, described in T. W. Greene and P. G. M. Wuts "Protective Groups in Organic Synthesis" 3rd Edition, June 1999, John Wiley & Sons Inc. For example, when the carbonyl group and the group X are protected as a 1,3-dioxolane group and as a tert-butyl carbamide, respectively, the protecting groups can be cleaved simultaneously using a suitable acid treatment.

In a preferred embodiment, of the method for the preparation of the compound of formula (I) the nitrosating step is preceded by a step of reacting a compound of formula (III) 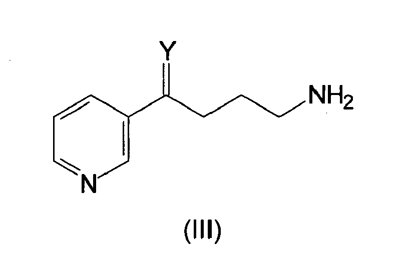 with a compound of formula (IV)
Z-W-X'
(IV)

=Y in formula (III) is as defined for formula (II) above.

X' in formula (IV) is as defined for formula (II) above.

W in formula (IV) is as defined for formula (II) above.

Z is a leaving group. The skilled artisan will readily be able to determine suitable leaving groups that can be employed in the coupling of the compound of formula (III) with the compound of formula (IV). Examples thereof include Cl, Br and I. In a preferred embodiment, Z is I.

The step of coupling the compound of formula (III) with the compound of formula (IV) can be carried out using any of the protocols that are commonly available for nucleophilic substitution reactions, wherein a leaving group of a first molecule is replaced by an incoming amine group of a second molecule. In a preferred embodiment, Z is halogen and the coupling is carried out in the presence of an equimolar amount of a base, such as cesium carbonate.

The compound of formula (III) can for example be obtained by the following three step sequence: Nicotine aldehyde is condensed with acrylonitrile, for example according to Stetter's procedure (H. Stetter et al., Org. Synth. (1980) 59, 53). The carbonyl group of the resulting nitrile is protected. This can, for example, be achieved by treatment with ethylene glycol and acid, such as p-toluenesulfonic acid, thereby yielding a 1,3-dioxolane group. The protected compound is then reduced, for example catalytically with H₂/Raney nickel as described by R. Soyka et al., J. Med. Chem. (1994) 37, 26.

A number of compounds falling under formula (IV) are commercially available, for example, 1-chloro-3-iodopropane, 3-iodopropionic acid and 1-chloro-4-iodobutane. Alternatively, compounds of formula (IV) can readily be synthesized using standard chemistry. For example, the amino group of a suitable aminoalcohol can be protected, for example using Boc-anhydride. Subsequently, the hydroxyl group can be converted into the desired leaving group, for example into iodide by reaction with iodine, imidazole and triphenylphosphine.

In order to induce sustained and specific antibodies a functionalized derivative of NNK is conjugated to a carrier providing T cell epitopes to activate T helper cells. The prior art antibodies were raised against conjugates obtained by derivatizing commercially available NNK. Derivatization was either carried out at the keto group of NNK, by a reaction on the pyridyl ring or by a reaction which resulted in loss of the nitroso group. Without wishing to be bound by theory, the superior properties of the present antibodies may be due to the fact that the chemical structure of NNK is essentially completely retained in the conjugates against which the antibodies are raised.

Accordingly, the present invention relates in a further aspect to a conjugate obtainable by coupling a compound of formula (I) to an immunogenic carrier.

A conjugate is to be understood as a hapten resembling NNK covalently linked to an immunogenic carrier. Linkage can take place via group X but also via any other functional group of the compound of formula (I) suitable to create a conjugate capable of inducing a T-cell response in a subject for the production of antibodies. In a preferred embodiment, the conjugate is obtainable by coupling the compound of formula (I) via group X to the immunogenic carrier.

An immunogenic carrier as used throughout the present invention is defined as a substance capable of inducing a T-cell response in a subject. Suitable substances are proteins, peptides, oligonucleotides or polymers. An immunogenic carrier typically has a molecular weight of at least 1000 Daltons, preferably more than 10000 Daltons. Carrier molecules often contain a reactive group to facilitate covalent conjugation to the hapten. The carboxylic acid group or amino group of amino acids or the sugar groups of glycoproteins are examples of reactive groups which can be used to this end. Carriers lacking such groups can be reacted with an appropriate chemical to produce a reactive group.

The presence of an immunogenic carrier increases the immunogenicity of the compound of formula (I). Polymeric immunogenic carriers can be natural or synthetic materials containing, for example, a primary and/or secondary amino group, an azido group or a carboxyl group. The carrier may be water soluble or insoluble.

In a preferred embodiment, the immunogenic carrier is a protein. A protein according to the present invention is defined as any high molecular mass compound consisting of one or more linear chains of the 20 amino acids of the genetic code joined by peptide bonds. The amino acids in the chains may be naturally modified by e.g. glycosylation, acetylation, phosphorylation, reduction and similar modifications which are well known in the art. Proteins can be obtained by isolation from a natural source or by recombinant means, all well known to the person skilled in the art. Immunogenicity of a candidate protein can be tested by immunizing a non-human subject with the protein and testing whether an antibody response is elicited.

Any one of a variety of immunogenic carrier proteins may be used for the conjugate of the present invention. Specific examples are albumins, such as bovine serum albumine (BSA); globulins; thyroglobulins; hemoglobins; hemocyanins (particularly keyhole limpet hemocyanin (KLH)); proteins extracted from ascaris, e.g. ascaris extracts such as those described in J. Immun. 111 (1973) 260-268, J. Immun. 122 (1979) 302-308, J. Immun. 98 (1967) 893-900, and Am. J. Physiol. 199 (1960) 575-578 or purified products thereof; polylysine; polyglutamic acid; lysine-glutamic acid copolymers; copolymers containing lysine or ornithine; etc.. Classes of suitable proteins include pili, outer membrane proteins and excreted toxins of pathogenic bacteria, nontoxic or "toxoid" forms of such excreted toxins, nontoxic proteins antigenically similar to bacterial toxins (cross-reacting materials or CRMs) and other proteins. Nonlimiting examples of bacterial toxoids contemplated for use in the present invention include tetanus toxin/toxoid, diphtheria toxin/toxoid, detoxified P. aeruginosa toxin A, cholera toxin/toxoid, pertussis toxin/toxoid and Clostridium perfringens exotoxins/toxoid. The toxoid forms of these bacterial toxins are preferred. The use of viral proteins (i.e. hepatitis B surface/core antigens; rotavirus VP 7 protein and respiratory syncytial virus F and G proteins) is also contemplated. The purified protein derivative of tuberculin (PPD) is considered to be particularly preferred for utilization in the "active" immunization scheme since (I) it does not induce a T-cell response itself (i.e. it is in effect a "T-cell hapten"), and yet behaves as a fully processed antigen and is recognized by T-cells as such; (2) it is known to be one of the most powerful hapten "carriers" in the linked recognition mode; and (3) it can be used in humans without further testing. CRMs include CRM₁₉₇, which is antigenically equivalent to diphtheria toxin (Pappenheimer et al., Immunochem., 9:891-906, 1972) and CRM₃₂₀₁, a genetically manipulated variant of pertussis toxin (Black et al., Science, 240:656-659, 1988). The use of immunogenic carrier proteins from non-mammalian sources, including keyhole limpet hemocyanin, horseshoe crab hemocyanin and plant edestin, is also within the scope of the invention.

In a preferred embodiment, the protein is diphtheria toxoid, ovalbumin, serum albumin, tetanus toxoid, keyhole limpet hemocyanin or CRM₁₉₇.

Examples of oligonucleotides and polymers that can be used as immunogenic carriers include CPG ODN conjugate (M. Hayashi et al., Biochemical and Biophysical Research Communications (2005) 329, 230-236).

In a further aspect, the present invention relates to a method for the preparation of the conjugate described above, comprising: (a) activating the immunogenic carrier; and (b) coupling the activated immunogenic carrier obtained in step (a) to the compound of formula (I).

The activation/coupling procedure can be carried out using standard protocols, such as the ester procedure described in Z. Grabarek et al., Anal. Biochem. (1990) 185, 131. Thus, in a preferred embodiment in step a) the immunogenic carrier is activated as a succinimid ester. To this end, the immunogenic carrier can, for example, be activated with a mixture of sulfo-N-hydroxy-succinimide and 1-ethyl-3-(3-dimethylaminopropyl) carbodiimide hydrochloride. The resulting succinimide ester is then reacted in step b) with a compound of the present invention wherein X is NH₂.

In another preferred embodiment in step a) the immunogenic carrier is activated as an o-acylisourea active intermediate using 1-cyclohexyl-3-(2-morpholinoethyl) carbodiimide. The resulting ester is then reacted in step b) with a compound of the present invention wherein X is NH₂.

In a further aspect, the present invention relates to a method for the preparation of the conjugate described above, comprising (a) activating group X of the compound of formula (I); and (b) coupling the activated compound obtained in step (a) to the immunogenic carrier.

For example, when X is COOH, the preparation of the conjugate can comprise: (a) activating the compound of formula (I) as a succinimid ester and (b) coupling the activated ester obtained in step (a) to the immunogenic carrier.

When X is NH₂ or SH, in step a) group X can be activated by reaction with a bifunctional reagent (such as sulfosuccinimidyl-4-(N-maleimidomethyl)cyclohexane-1-carboxylate (sulfo-SMCC); 3,3'-dithiobis(sulfosuccinimidylpropionate) (DTSSP); or succinimidyl-6-(3-[2-pyridyldithio]-propionamido)hexanoate (LC-SPDP)) in order to conjugate the compound of formula (I) to the immunogenic carrier. Preferably, when X is NH₂ or SH, X is activated in step a) by reaction with a maleimide.

When, in the compound of formula (I), X is halogen, in particular I, the step of activating group X can be omitted.

For the preparation of the conjugate the skilled artisan will readily be able to determine suitable conjugation methods, for example, to be found in Hermanson, G. T. Bioconjugate Techniques; 1st ed.; Academic Press: San Diego, California, 1996; Vol. 1.

When the immunogenic carrier is a protein, the methods for the preparation of the conjugate preferably further comprise the step of reducing and alkylating the protein prior to step a). To this end, the protein can, for example, be subjected to a treatment with dithio-1,4-threitol, followed by iodoacetamide or iodoacetic acid. The purpose of this reduction/alkylation is to at least partially denature the protein, in order to remove secondary and tertiary structure arising, for example, from the presence of disulfide bridges thus increasing the number of exposed reactive groups.

The present invention also relates to an antibody. The present antibody specifically binds to at least one of: the compounds of the present invention, optionally bound to an immunogenic carrier, NNK and metabolites thereof (preferably non-detoxified metabolites thereof, in particular NNAL). In the embodiment of the antibody which specifically binds to the compound of the present invention bound to an immunogenic carrier and thereby forming the conjugates of the invention, the antibody specifically binds to epitopes formed by the hapten, rather than to the immunogenic carrier.

In the context of the present invention, in non-detoxified metabolites of NNK the pyridyl group is not N-oxidized. Non-detoxified metabolites of NNK include NNAL (4-(methylnitrosamino)-1-(3-pyridyl)-1-butanol), HPB (4-hydroxy-1-(3-pyridyl)-1-butanone), PB (1-(3-pyridyl)-1-butanediol), keto acid (1-(3-pyridyl)-1-butanone-4 carboxylic acid) and PBCA (1-(3-pyridyl)-1-butanol-4 carboxylic acid). In the context of the present invention, in detoxified metabolites of NNK the pyridyl group is N-oxidized, glucoronidated or the keto group is substituted. Detoxified metabolites of NNK include NNK-N-Ox (4-(methylnitrosamino)-1-(3-pyridyl-N-oxide)-1-butanone), NNAL-N-Ox (4-(methylnitrosamino)-1-(3-pyridyl-N-oxide)-1-butanol) and NNAL-O-Gluc (4-(methylnitrosamino)-1-(3-pyridyl)but1-yl)-β-O-D-glucoronic acid).

In the embodiment of the antibody the binding of said antibody to NNK, NNAL and non-detoxified metabolites of NNK or NNAL is stronger than to derivatives of NNK and/or NNAL having an N-oxidized pyridyl group or glucoronidated species. Further, binding to NNK and NNAL can be stronger than to other non-detoxified metabolites.

Preferably, the antibody of the present invention binds to NNK with a dissociation constant K_{D} of less than 5 µM, preferably less than 500 nM, more preferably less than 100 nM.

In the context of the present invention, it is further preferred that the antibody binds both NNK and NNAL. In particular, it is preferred that the antibody binds stronger to NNK and non-detoxified metabolites or derivatives than to detoxified metabolites or derivatives thereof, i.e. derivatives wherein the nitrogen of the pyridyl moiety is oxidized or which are glucoronidated, for example binding to NNK versus binding to NNK-N-Ox or binding to NNAL versus binding to NNAL-N-Ox. Preferably, specific binding of the antibody to NNK and/or said non-detoxified metabolites thereof is defined as an at least 10, more preferably at least 100, even more preferably at least 1000, even more preferably at least 10,000, and even more preferably at least 100,000 higher affinity of the antibody to NNK and/or non-detoxified metabolites thereof than to detoxified derivatives of NNK having an N-oxidized pyridyl group. It is further preferred that the antibody does not bind to detoxified metabolites of NNK. The relative binding can be measured by the competition ELISA assay described in example 4.

The antibody binds to the compound of any one of claims 1 to 3 bound to an immunogenic carrier with a dissociation constant K_{D} of less than 100 nM, preferably less than 75 nM, even more preferably less than 50 nM. The dissociation constant can be measured by the surface plasmon resonance assay described in example 4.

The antibody of the invention does not cross-react with epitopes not related to NNK or derivatives thereof. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest but do not or do not essentially bind to any of the other epitopes are considered specific for the epitope of interest and thus to be antibodies in accordance with this invention. Corresponding methods are described e.g. in Harlow and Lane, "Antibodies, A Laboratory Manual", Cold Spring Harbor Laboratory Press, 1988 and Harlow and Lane "Using Antibodies: A Laboratory Manual" Cold Spring Harbor Laboratory Press, 1999. Not binding essentially is to be understood as binding with at least 100, more preferably at least 1000, even more preferably at least 10000, and most preferably at least 100,000 times lower affinity than NNK.

The antibody of the present invention can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

In a further aspect, the present invention relates to an antibody wherein CDR1 of V_{H} has the sequence of [SEQ ID NO: 3] or CDR2 of V_{H} has the sequence of [SEQ ID NO: 4] or CDR3 of V_{H} has the sequence of [SEQ ID NO: 5] or CDR1 of V_{L} has the sequence of [SEQ ID NO: 6] or CDR2 of V_{L} has the sequence of [SEQ ID NO: 7] or CDR3 of V_{L} has the sequence of [SEQ ID NO: 8]. Any combination of these CDR regions producing an antibody capable of exerting the desired functions, i.e. binding NNK and/or a metabolite thereof, lie within the scope of the present invention. For example, the antibody can comprise CDR1, CDR2 and CDR3 of V_{L} or CDR1, CDR2 and CDR3 of V_{H}. Preferably, the antibody of the present invention comprises all six CDR regions.

Preferably, in the antibody CDR1 of V_{H} has the sequence of [SEQ ID NO: 11] or CDR2 of VH has the sequence of [SEQ ID NO: 12] or CDR3 of V_{H} has the sequence of [SEQ ID NO: 13] or CDR1 of V_{L} has the sequence of [SEQ ID NO: 14] or CDR2 of V_{L} has the sequence of [SEQ ID NO: 15] or CDR3 of V_{L} has the sequence of [SEQ ID NO: 16]. Any combination of these CDR regions producing an antibody capable of exerting the desired functions, i.e. binding NNK and/or a metabolite thereof, lie within the scope of the present invention. For example, the antibody can comprise CDR1, CDR2 and CDR3 of V_{L} or CDR1, CDR2 and CDR3 of V_{H}. Preferably, the antibody of the present invention comprises all six CDR regions.

In a preferred embodiment, the V_{H} and V_{L} amino acid sequences of the antibody have at least 85%, preferably at least 90%, more preferably at least 95%, even more preferably at least 98% sequence identity with P9D5 or P7H3 having V_{H} and V_{L} amino acid sequences as depicted in SEQ ID NOs: 1 and 2 for P9D5 and SEQ ID NOs: 9 and 10 for P7H3. Most preferably, the antibody is P9D5 or P7H3.

In another embodiment, the present invention relates to an antibody wherein CDR1 of V_{H} has the sequence of [SEQ ID NO: 3], CDR2 of V_{H} has the sequence of [SEQ ID NO: 4], CDR3 of V_{H} has the sequence of [SEQ ID NO: 5], CDR1 of V_{L} has the sequence of [SEQ ID NO: 6], CDR2 of V_{L} has the sequence of [SEQ ID NO: 7] and CDR3 of V_{L} has the sequence of [SEQ ID NO: 8].

In a further embodiment, the present invention relates to an antibody wherein CDR1 of V_{H} has the sequence of [SEQ ID NO: 11], CDR2 of V_{H} has the sequence of [SEQ ID NO: 12], CDR3 of V_{H} has the sequence of [SEQ ID NO: 13], CDR1 of V_{L} has the sequence of [SEQ ID NO: 14], CDR2 of V_{L} has the sequence of [SEQ ID NO: 15] and CDR3 of V_{L} has the sequence of [SEQ ID NO: 16].

The antibody of the invention also includes embodiments such as chimeric, single chain and humanized antibodies, as well as antibody fragments, like, inter alia, Fab fragments. Antibody fragments or derivatives further comprise F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for haptens and/or conjugates of this invention. Also, transgenic animals may be used to express humanized antibodies specific for haptens and/or conjugates of this invention. Most preferably, the antibody of this invention is a monoclonal antibody. For the preparation of monoclonal antibodies, any technique which provides antibodies produced by continuous cell line cultures can be used. Examples for such techniques include the hybridoma technique (Köhler and Milstein, Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of hapten or conjugate of the invention (Schier, Human Antibodies Hybridomas 7 (1996), 97-105; Malmborg, J. Immunol. Methods 183 (1995), 7-13). The antibody according to the present invention the DNA encoding it can be sequenced providing for the information to recombinantly produce the antibody of the invention in small or large scale. Methods of the production of a recombinant antibody are known to the person skilled in the art and can be found in Harlow E. et al, Antibodies: A Laboratory Manual; Cold Spring Harbor Laboratory Press, 1988. The present invention also relates to a method for producing the present antibodies comprising immunizing a non-human animal with the conjugate of the present invention. Methods for producing antibodies are well known to the person skilled in the art and are briefly described herein.

The antibody of the invention has several advantages as compared to the antibodies produced in the prior art. Compared to the antibody described by Talbot, B. et al., Arch. Toxicol. (1990) 64, 360-364, the antibodies of the present invention described in the examples below recognize NNK and NNAL (i.e.1 two of the most carcinogenic species of the metabolic cascade) and optionally further non-detoxified metabolites thereof, but not the detoxified N-oxide derivatives or glucorinated derivatives. The present antibody can thus be applied to detect the carcinogenic NNK and carcinogenic metabolites thereof and as vaccine as both described below.

The present antibody could also be used in a pharmaceutical composition. For example, the present antibody directed against NNK or a metabolite thereof could be administered for treating or preventing a disorder associated with tobacco specific nitrosamine exposure.

Tobacco specific nitrosamines include NNK. Tobacco specific nitrosamine exposure can, for example, arise from exposure to tobacco smoke (main stream or side stream, i.e. active or passive smoking) as well as other forms of tobacco consumption.

The experimental data presented below shows that the present antibodies are capable of interfering with the cellular uptake, transepithelial transport and metabolism of both NNK and its major metabolite NNAL and inhibit NNK induced SCLC cells proliferation, mechanisms, which are likely to also have long-term effects.

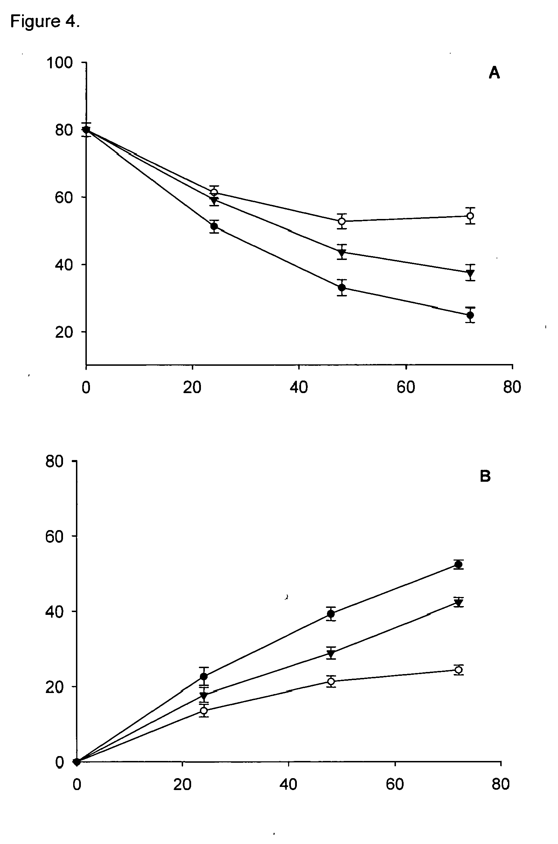

In particular, antibodies falling within the scope of the present invention are capable of binding NNK for prolonged time-periods (Fig. 4A). Without wishing to be bound by theory, the following mechanisms may be relevant for the present medical applications. *In vivo*, antibodies in the lung may modulate the cellular uptake and the rate of NNK metabolism both at the level of the pro-carcinogen and its major metabolite NNAL. In contrast to NNK and NNAL, preferred antibodies do not bind to the detoxified metabolites resulting from pyridine N-oxidation. Thus, these preferred antibodies do not interfere with the excretion of these end-point metabolites, and are not blocked by binding to these innocuous molecules.

As shown below in the examples, the present antibodies can prevent the NNK-mediated mitogenic stimulation of a human small cell lung carcinoma cell line upon binding to the α7-nicotinic receptor. Thus, sequestration of NNK by antibody in the interstitium may not only prevent DNA adduct formation but also NNK-mediated proliferation of SCLC. This is of particular interest since it has been suggested that progression of SCLC in smokers is largely due to chronic and selective mitogenic stimulation of bronchial cells by NNK binding to α7 nAChR. A similar effect of NNK has also been observed in colon carcinoma cells.

In addition, several other mechanisms can translate into long-term effects. (i) Antibodies may redistribute carcinogen from sensitive lung mucosa towards organs that cope better with its metabolic turnover (i.e., detoxification) and are therefore less sensitive. For instance, the liver is much less sensitive to NNK-induced carcinogenesis because of its more efficient DNA repair mechanisms compared to lung tissue in which repair enzymes are easily depleted for days, even after a single carcinogen administration. NNK is a systemic carcinogen, which after uptake (irrespective of the route) induces tumors predominantly in the lung, most probably by extensive first-pass metabolism in the sensitive lung tissue. Therefore, antibody-mediated redistribution via blood and lymphatic circulation or receptor-mediated transport to the interstitial space of the liver, may shift carcinogen metabolism and adduct formation predominantly towards this organ. Therefore, even trapping of NNK in the bloodstream can be expected to decrease the risk of lung cancer. (ii) Antibodies may also confer protection simply by preventing high peak intracellular concentrations of adduct forming species, which may overload DNA repair mechanisms. (iii) Similarly, by preventing high-peak intracellular concentrations, antibodies may modulate the rate of formation of proximate and ultimate phase 1 carcinogen species (e.g. by inhibition of P450 induction) and reduce carcinogenesis by restoring a more favorable balance between phase 1 and phase 2 metabolism. (iv) The most direct long-term effect of antibodies on NNK activity may result from inhibition of NNK-mediated proliferation of SCLC, which is considered one of the most important mechanisms of clinical carcinogenesis in the lung. The experimental data presented below shows that antibodies can reduce stimulation of first SCLC progenitor cells by blunting local peak concentrations of NNK, and thus prevent or delay tumor growth.

In addition, the present invention relates to a pharmaceutical composition comprising the conjugate of the present invention.

In accordance with the present invention, the term "pharmaceutical composition" relates to a composition for administration to a patient, preferably a human patient. The pharmaceutical composition of the invention comprises the compounds recited above, alone or in combination. It may, optionally, comprise further molecules capable of altering the characteristics of the compounds of the invention thereby, for example, stabilizing, modulating and/or activating their function. The composition may be in solid, liquid or gaseous form and may be, *inter alia*, in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include phosphate buffered saline solutions, water, emulsions, such as oil/water emulsions, various types of wetting agents, sterile solutions, organic solvents, including dimethylsulfoxide, etc. Compositions comprising such carriers can be formulated by well known conventional methods. The term "parenteral" as used herein refers to modes of administration which include intravenous, intramuscular, intraperitoneal, intrasternal, subcutaneous and intraarticular injection and infusion. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

In a preferred embodiment, the pharmaceutical composition is a vaccine. For example, in vivo, antibodies induced by hapten-conjugate vaccines are expected to substantially reduce cancer risk in people that cannot stop exposing themselves chronically to this carcinogen. The conjugate vaccine or the antibody vaccine of the invention may be in soluble or microparticular form, or may be incorporated into microspheres or microvesicles, including liposomes. Although various routes of vaccine administration including, for example, intramuscular, subcutaneous, intraperitoneal and intraarterial are contemplated, the preferred route is intramuscular administration. In a preferred embodiment, the dosage of the conjugate administered will range from about 10 µg to about 50 µg In a more preferred embodiment, the amount administered will be between about 20 µg and about 40 µg. In a most preferred embodiment, the amount administered is about 25 µg. Greater doses may be administered on the basis of body weight. The exact dosage can be determined by routine dose/response protocols known to one of ordinary skill in the art.

The vaccine of the invention may be administered to warm-blooded mammals of any age and are adapted to induce active immunization in young mammals, particularly humans, against NKK or NNAL exposure. As a childhood vaccine, the conjugate is administered at about 2 to 4 months of age. Typically, two booster injections of between about 10 µg and about 25 µg are administered at about 2 and again about 13 months after the initial injection. Alternatively, three booster injections are given at 2, 4 and 16 months after the initial injection.

The present invention further relates to the conjugate of the invention for treating and/or preventing disorders associated with tobacco specific nitrosamine exposure. In addition, the present invention relates to the antibody of the invention for treating and/or preventing disorders associated with tobacco specific nitrosamine exposure.

The effects as described above for the antibody of the present invention concerning long-term effects apply to preferred embodiments of the conjugate of the invention as a pharmaceutical composition or vaccine eliciting antibodies directed against NNK, NNAL and non-detoxified metabolites thereof.

In a preferred embodiment of the conjugate or the antibody of the present invention for the treatment of a disorder associated with tobacco specific nitrosamine exposure, the disorder to be treated is cancer, in particular lung cancer, pancreatic cancer, cancer of the oespohagus and oral cancer and cancers of the respiratory tract in general. Examples for specific cancers attributed to the exposure to NNK or its carcinogenic metabolites are small cell lung cancer, adenocarcinoma in lungs or oral tumours. In general, any disorder or disease related to smoking is envisaged in the use of the present invention. Thereby, disorders or diseases resulting form active self-exposure fall within the scope of the present invention as well as those arising from passive exposure to tobacco smoke, in particular disorders or diseases related to nitrosamines (e.g. NKK and its metabolites).

In a different aspect, the present invention relates to a method for the detection of antibodies specific for NNK or a non-detoxified metabolite thereof, comprising subjecting a sample from a subject to the conjugate of any one of claims 8 to 11 and detecting binding of the conjugate to the antibody. Binding can be detected by any of the methods described herein.

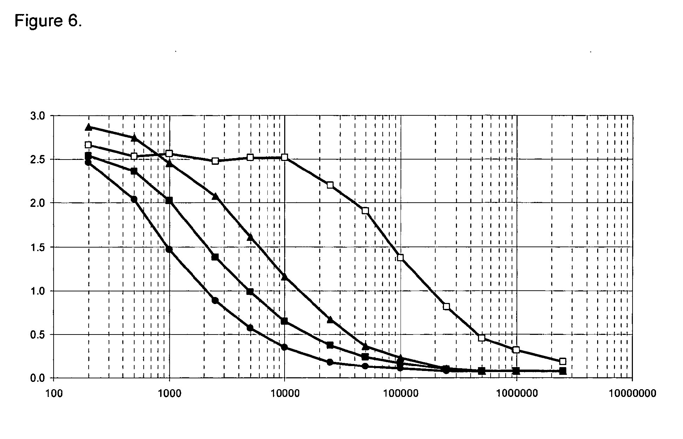

In some embodiments, the sample is contacted with the conjugate immobilized onto a substrate under conditions suitable to distinguish specific from non-specific binding which conditions can readily be determined by a person skilled in the art (cf., for example, the indirect ELISA assay described in example 4 and illustrated in Figure 6). The substrate is then washed to remove any unbound antibody. The antibody under assessment is also contacted (either subsequently or simultaneously with its contact with the conjugate) with a labeled antibody specific for the antibody under assessment under conditions effective to cause the labeled antibody to bind the antibody under assessment. The substrate is then washed to remove any unbound antibody and the presence and/or concentration of the antibody under assessment is indicated by the presence and/or strength of the label signal. Other assay method, including other types of direct and indirect assays as well as competitive assays may be used.

In another aspect, the present invention relates to a method of detecting NNK or NNAL or another non-detoxified metabolite thereof, comprising subjecting a sample to the antibody of the present invention and detecting binding of the antibody to NNK or a non-detoxified metabolite thereof. In particular, the antibodies of the present invention can be used to detect TSNAs, preferably NNK, NNAL and non-detoxified metabolites thereof and their adducts to proteins or DNA. As explained in the background section, these adducts can contain the pyridyloxobutyl moiety originating from NNK.

In the present method of detecting NNK or NNAL or another non-detoxified metabolite thereof, the antibodies of the present invention can be used, for example, for the immunoprecipitation, affinity purification and immunolocalization of the haptens or conjugates of the invention as well as for the monitoring of the presence and amount of such compounds, molecules, metabolites or adducts of TSNA in biological or environmental samples.

Suitable methods to be applied are known to the person skilled in the art. Antibody-based detection allows the detection, quantification and/or localisation of antigens by means of antibody binding. Methods for the quantification of an antigen are e.g. radioimmunoassays (RIA) and enzyme-linked immunosorbent assays (ELISA). Another suitable method according to the present invention comprises immunoprecipitation, e.g. by using (magnetic) beads coated with the antibodies of the invention. The principles and applications of these methods are known to the skilled person and are described e.g. in Harlow E. et al, Antibodies: A Laboratory Manual; Cold Spring Harbor Laboratory Press, 1988.

In some embodiments, the antibody is labeled directly or indirectly to allow detection of analyte in a sample. For example, the labeled antibody is combined with the sample, and the labeled antibody-analyte complex is detected. The antibody is, for example, labeled during antibody production, or a label is conjugated to the antibody by joining it to the antibody, either covalently or non-covalently. A wide variety of labels and conjugation techniques are known and are reported extensively in both the scientific and patent literature. Any suitable label and any suitable conjugation technique may be used. Suitable labels include radioactive molecules, enzymes, substrates, cofactors, inhibitors, fluorescent substances, chemiluminescent substances, chromogenic substances, magnetic particles, and the like. The detectable group can be any material having a detectable physical or chemical or other property. Such detectable labels have been well developed and, in general, any label useful in such methods can be applied in the context of the present invention. Thus, examples of labels include any composition detectable by spectroscopic, photochemical, biochemical, immunochemical, electrical, optical or chemical means.

Useful fluorescent substances include fluorescein and its derivatives (e.g. isothiocyanate), Texas red, rhodamine and its derivatives, dansyl, umbelliferone and the like. Chemiluminescent compounds include luciferin, and 2,3-dihydrophthalazinediones, e.g., luminol. Useful radiolabels include 3H, 1251, 35S, 14C, or 32P. Useful enzymes include hydrolases, particularly phosphatases, esterases and glycosidases, or oxidoreductases, particularly peroxidases, LacZ, CAT, horseradish peroxidase, alkaline phosphatase and others, commonly used as detectable enzymes, either in an EIA or in an ELISA. Furthermore, colorimetric labels such as e.g. colloidal gold or colored glass or plastic (e.g. polystyrene, polypropylene, latex, etc.) beads can be used. The label may be coupled directly or indirectly to the desired component of the assay according to methods well known in the art. As indicated above, a wide variety of labels may be used, with the choice of label depending on the sensitivity required, ease of conjugation of the compound, stability requirements, available instrumentation, and disposal provisions. Nonradioactive labels are often attached by indirect means. In one embodiment, a molecule having a known binding affinity is covalently bound to the antibody. The covalently bound molecule then binds to another molecule, which is either inherently detectable or covalently bound to a signal system, such as a detectable enzyme, a fluorescent compound, or a chemiluminescent compound. Any pair of molecules having such binding affinities that will function as part of this assay can be used. Alternatively, any haptenic or antigenic compound can be used in combination with an antibody. In some embodiments, the antibodies are conjugated directly to signal generating compounds, e.g., by conjugation with an enzyme or fluorophore.

Furthermore, in some embodiments, the antibody is labeled indirectly by reaction with labeled substances that have an affinity for immunoglobulin, such as protein A or G or secondary antibodies. In some embodiments, the antibody is conjugated with a second substance and detected with a labeled third substance having an affinity for the second substance conjugated to the antibody.

These and other methods of labeling antibodies and assay conjugates are well known to those skilled in the art.

A highly sensitive assay is provided employing an antibody specific for one or more of NNK and metabolites thereof (preferably non-detoxified metabolites, in particular NNAL). The assay is useful for detecting the presence or amount of one or more of the above substances in one or more samples. A preferred sample is tobacco or a tobacco product or preparation. A preferred assay is an immunoassay that employs an antibody specific for one or more of NNK and metabolites thereof (preferably non-detoxified metabolites, in particular NNAL) thereof, such as the antibodies described above. The assay provides antibodies with affinity and specificity for NNK and/or metabolites thereof (preferably non-detoxified metabolites, in particular NNAL), preferably while lacking significant crossreactivity for other TSNAs. In one embodiment, the methods and assays use antibodies having much lower binding affinity, or crossreactivity, with nicotine than with TSNAs. In embodiments involving immunoassays, the antibodies may be employed in any heterogeneous or homogeneous, sandwich or competitive immunoassay for the detection of one or more of the above substances. Any suitable method or milieu may be used to perform the immunoassay, including but not limited to coated tubes, magnetic beads, lateral flow strips, agglutination assays, turbimetric assays and radioimmunoassays. Assays include quantitative assays and qualitative assays, e.g. around a cutoff limit. In some embodiments, assays are read with suitable instrumentation or visually by eye depending on desired accuracy.

In some embodiments, the labeled antibody is coupled to a solid phase to facilitate detection. Any suitable method of labeling or coupling may be used. Methods for coupling antibodies to solid phases are well known to those skilled in the art. In accordance with the immunoassay method, the sample containing or suspected of containing TSNAs or derivatives is reacted with the antibody for a sufficient amount of time under conditions that promote the binding of antibody to one or more TSNAs or derivatives in the sample. It will be understood by those skilled in the art that the immunoassay reagents and sample may be reacted in different combinations and orders. A physical means is employed in some embodiments to separate reagents bound to a solid phase from unbound reagents. Examples of such means include, but are not limited to filtration of particles, decantation of reaction solutions from coated tubes or wells, magnetic separation, capillary action, and other means known to those skilled in the art. It will also be understood that a separate washing of the solid phase may be included in the method. Any solid phase that will allow immobilization may be used. It will be understood by those skilled in the art that examples of solid phases include latex, polystyrene, polyethylene, polypropylene, polycarbonate, nitrocellulose or any other solid material in the shape of test tubes, microtiter plates, beads, microparticles, dip-sticks, test strips, or the like. Other solid phases include, but are not limited to, glass beads, glass test tubes and any other appropriate shape made of glass or plastic. Preferably, the solid phase is a nitrocellulose strip. After reaction, the existence, concentration, or both of the analyte is determined by the signal generated by a label. The presence, absence, intensity, or location of the signal may be an indicator.

In one embodiment, the sample is contacted with the antibody immobilized onto a substrate under conditions effective to cause the antibody to bind analyte in the sample. The bound antibody is also contacted (either subsequently or simultaneously with its contact with the sample) with unbound antibody that is labeled under conditions effective to cause the labeled antibodies to bind the analyte that has already bound to the fixed antibody. The substrate is then washed to remove any unbound antibody and the presence and/or concentration of the analyte is indicated by the presence and/or strength of the label signal.

In a different embodiment involving a direct assay, the sample is placed under conditions effective to cause any analyte in the sample to become fixed on a substrate. The substrate is then contacted with labeled antibody under conditions effective to cause binding of the antibody to any bound analyte. The substrate is then washed to remove any unbound antibody and the presence and/or concentration of the analyte is indicated by the presence and/or strength of the label signal. In another embodiment, involving a competitive assay, antibodies that bind an analyte are bound to a substrate. The sample to be tested is contacted with the substrate together with analyte bound to a label. The substrate is then washed to remove any unbound analyte and the presence and/or concentration of the analyte is indicated by a weakening of the signal as compared to the signal strength when no sample is present. The foregoing are simply examples of assays and any suitable assay method may be used, including other types of direct and indirect assays as well as competitive assays.

It will be understood that the assay is not limited to embodiments in which a label is attached to the antibody. Labels may be used in any part of the assay that will result in a signal that may be interpreted appropriately. In some embodiments, a binding molecule specific for the antibody of the invention, such as an antibody, is labeled and the presence of a bound antibody of the invention is detected indirectly. In other embodiments, such as some competitive assays, the label is attached to a molecule that competes with analyte in the sample for binding to an antibody. In some embodiments, no label is used and the presence, location, or concentration of antibody is determined by other means.

Examples of known methods include, but are not limited to immunoblotting, western analysis, gel-mobility shift assays, fluorescent in situ hybridization analysis (FISH), tracking of radioactive or bioluminescent markers, nuclear magnetic resonance, electron paramagnetic resonance, stopped-flow spectroscopy, column chromatography, capillary electrophoresis, or other methods which track a molecule based upon an alteration in size and/or charge.

For example, where the label is a radioactive label, means for detection include a scintillation counter or photographic film as in autoradiography. After exciting the fluorochrome of fluorescent labels with the appropriate wavelength of light, the resulting fluorescence may be detected by e.g. microscopy, visual inspection, via photographic film, by the use of electronic detectors such as charge coupled devices (CCDs) or photomultipliers and the like. Similarly, enzymatic labels are detected by providing appropriate substrates for the enzyme and detecting the resulting reaction product. Finally, simple colorimetric labels may be detected simply by observing the color associated with the label. Thus, in various dipstick assays, conjugated gold often appears pink, while various conjugated beads appear the color of the bead. Preferred detection methods include a direct or indirect enzyme-linked immunosorbent assay (ELISA) using a secondary antibody such as a peroxidase- conjugated goat anti-mouse antibody, a competitive ELISA using an immobilized goat anti-mouse antibody, a mouse antibody reactive with the analyte, and an analyte conjugated to a label, or a direct or indirect immunofluorescence assay using a secondary antibody such as a fluorescein isothiocyanate (FITC)-conjugated goat anti-mouse antibody. In some embodiments, the concentration of one or more of the above defined TSNAs in the sample is determined either visually and without the need for analytical instrumentation - for example, by comparing the intensity of the color produced by the sample to a standard such as a color card - or by using instrumentation such as a spectrophotometer or reflectometer. In some embodiments, the resulting reaction mixture or combination of antibody and sample is prepared in a solution that optimizes antibody-analyte binding kinetics. An appropriate solution is an aqueous solution or a buffer. The solution is preferably provided under conditions that will promote specific binding, minimize nonspecific binding, solubilize analyte, stabilize and preserve reagent reactivity, and may contain buffers, detergents, solvents, salts, chelators, proteins, polymers, carbohydrates, sugars, and other substances known to those skilled in the art.

In some embodiments, the antibodies provided herein are used to detect targets extracted into solution from a solid material. For example, a solid sample can be extracted with an aqueous liquid, an organic solvent or a critical fluid and the resulting supernatant can be contacted with the antibody. Examples of solid samples include tobacco plants and parts thereof such as leaves, stems and roots, processed tobacco, and tobacco products. The reaction mixture solution is reacted for a sufficient amount of time to allow the antibody to react and bind to the analyte to form an antibody-analyte complex. The shortest amount of reaction time that results in binding is desired to minimize the time required to complete the assay. One appropriate reaction time period for an immunochromatographic strip test is less than or equal to 20 minutes or between approximately one minute and 20 minutes. A reaction time of less than five minutes is preferred. Most preferably, the reaction time is less than three minutes. By optimizing the reagents, binding may be substantially completed as the reagents are combined. The reaction is performed at any temperature at which it will occur as desired. This is often the range of temperatures at which the reagents do not degrade or become inactivated. A temperature between approximately 4°C and 37°C is preferred. The most preferred reaction temperature is ambient or room temperature (approximately 25°C).

Strip tests are composed of multiple porous components, membranes and filters through which liquid sample is drawn by capillary action. Analyte in the sample reacts with the test reagents contained within the test strip as it traverses the length of the strip. In one embodiment, in which the goal is to detect an analyte in tobacco, the tobacco is ground into a powder and the analyte extracted from the powder with a liquid that is then separated from the solid material and assayed using the test.

The liquid is applied to a chromatographic strip, and the analyte migrates toward the distal end of the strip. As it migrates down the strip, the analyte reacts with reagents, e.g. antibodies, applied to or immobilized on the strip causing a detectable signal product. Detection of the signal indicates the presence of the analyte in the sample. In one preferred embodiment, the assay detects a total concentration of NNK and metabolites thereof (preferably non-detoxified metabolites, in particular NNAL) thereof in a sample. In one embodiment, the results of the assay are compared against the results using one or more standards containing known concentrations of the respective substances, wherein the individual TSNAs may be present in relative concentrations that approximate those in tobacco or other materials to be tested. Optionally, multiple standard solutions are used to prepare a calibration curve. Optionally, tests of individual TSNAs, e.g. NNK or metabolites thereof (preferably non-detoxified metabolites, in particular NNAL), are employed as a follow-up test to verify that the proportions are correct, and other proportions are used to develop standards if appropriate for a given sample.

In the present method, NNK or a metabolite thereof (preferably a non-detoxified metabolite, in particular NNAL) is detected in a sample obtained from a subject. The subject is preferably a mammal, e.g. a dog, a cat, a rodent, a horse, a cow, a pig or a primate. It is most preferred that the subject is a human.

Figures:

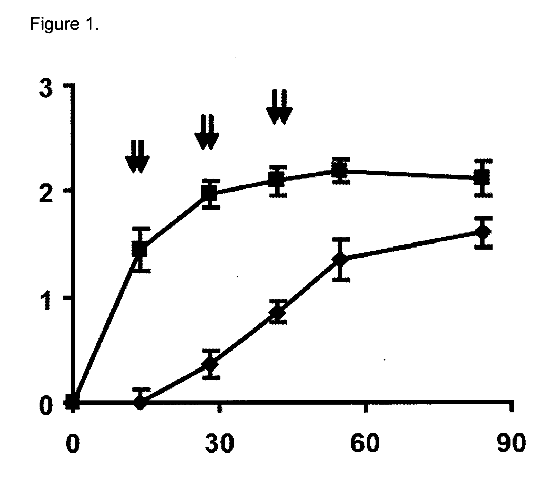

Figure 1: Reactivity of anti-[NNK-C2]-DT sera with coated [NNK-C2]-DT (upper curve) or [NNK-C2]-OVA (lower curve). Mice were primed with an ip. injection and boosted by gastric intubation. Serum dilution 1/1500. Mean ± SD of reactivity of two duplicate experiments after subtraction of background reactivity against irrelevant antigen (DT 0.084 ± 0.05 or OVA 0.077 ± 0.01). The x-axis shows days after first injections, the y-axis shows net O:D: (405 nm).

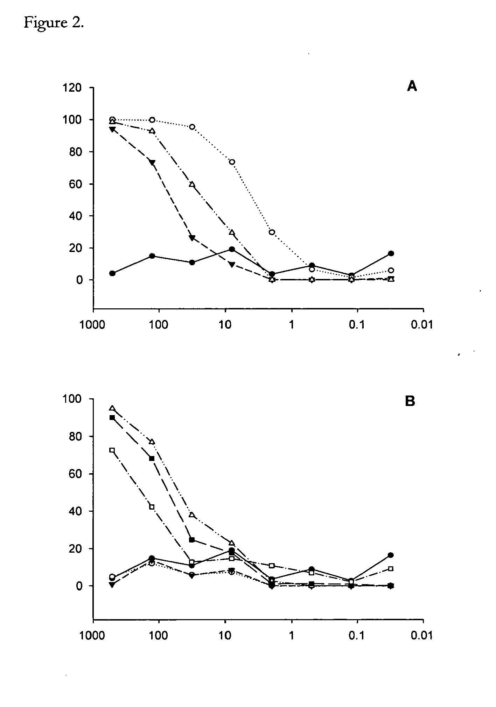

Figure 2: Inhibition of binding of P9D5 (1:1000 dilution) to immobilized [NNK-C2]-OVA by soluble NNK, its derivates and metabolites (n=5). Figure 2A: solid circle: no competitor, open circle: NNK-C2, solid triangle: NNK, open triangle: NNAL. The x-axis shows competitor concentration (µM), the y-axis shows level of competition (%). Figure 2B: solid circle: no competitor, open circle: NNK-N-Ox, solid triangle: NNAL-N-Ox, open triangle: HPB, solid square: PB, open square: PBCA. The x-axis shows competitor concentration (µM), the y-axis shows level of competition (%).

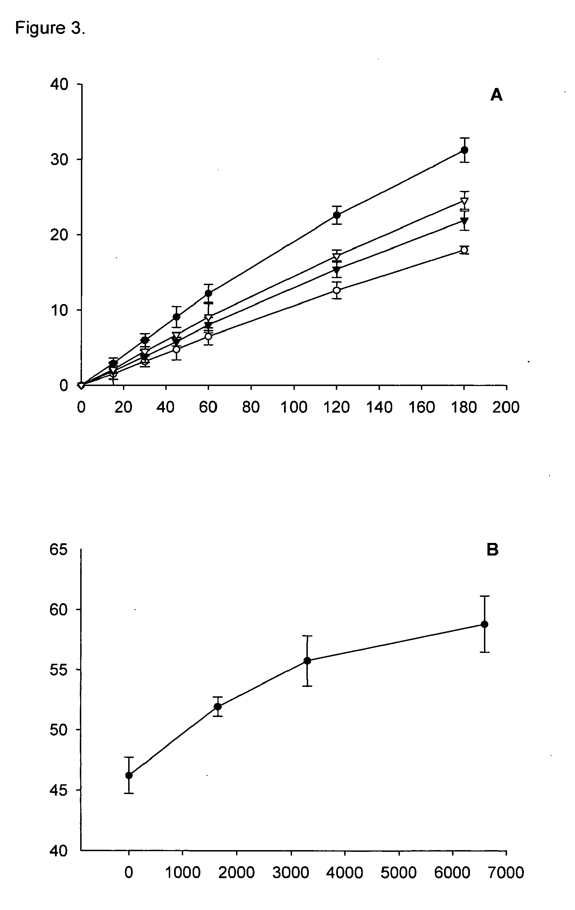

Figure 3: Influence of apical NNK antibody P9D5 (1650-6600 nM or 250-1000 pmol or 0.25-1mg/ml) on (A) the cumulative apical to basolateral absorptive transport of 130 nM [5-³H]NNK (20 pmol) (1 mg/ml mAb = 6600 nM); (B) and on the apical recovery of 130 nM [5³H]NNK (20 pmol) after 3 h of incubation with increasing concentrations of P9D5 antibody. Results are expressed in percentage of the total amount of [5³H]NNK initially administered at time 0 ± SD (n = 3). Figure 3A: solid circle: control mAb (6600 nM), open circle: P9D5 (6600 nM), solid triangle: P9D5 (3300 nM), open triangle P9D5 (1650 nM).The x-axis shows time (min), the y-axis shows transport transport of NNK (%). Figure 3B: solid circle: P9D5, open circle: control mAb. The x-axis shows time (min), the y-axis shows transport transport of NNK (%). The x-axis shows specific antibody in apical compartment (nM), the y-axis shows recovery of apical NNK after 3h (%).
Figure 4: Influence of the NNK antibody P9D5 (2700 - 5300 nM or 660-1320 pmol or 0.4-0.8 mg/ml) on (**A**) the recovery of co-administered [5-³H]NNK (80 nM, 20 pmol) in supernatant of Calu-3 cells (collagen-coated 96-well plates) incubated for 24-72 h; (**B**) and on NNAL levels in supernatant of Calu-3 cells (collagen-coated 96-well plates) incubated for 24-72 h with [5-³H]NNK. Results are expressed as the average cumulative concentration of NNAL in supernatants ± SD (n = 3). Figure 4A: solid circle: control mAb (5300 nM), open circle: P9D5 (5300 nM), solid triangle: P9D5 (2700 nM). The x-axis shows time (h), the y-axis shows concentration NNK (nM). Figure 4B: P9D5 (5300 nM), solid triangle: P9D5 (2700 nM). The x-axis shows time (h), the y-axis shows concentration NNAL (nM).

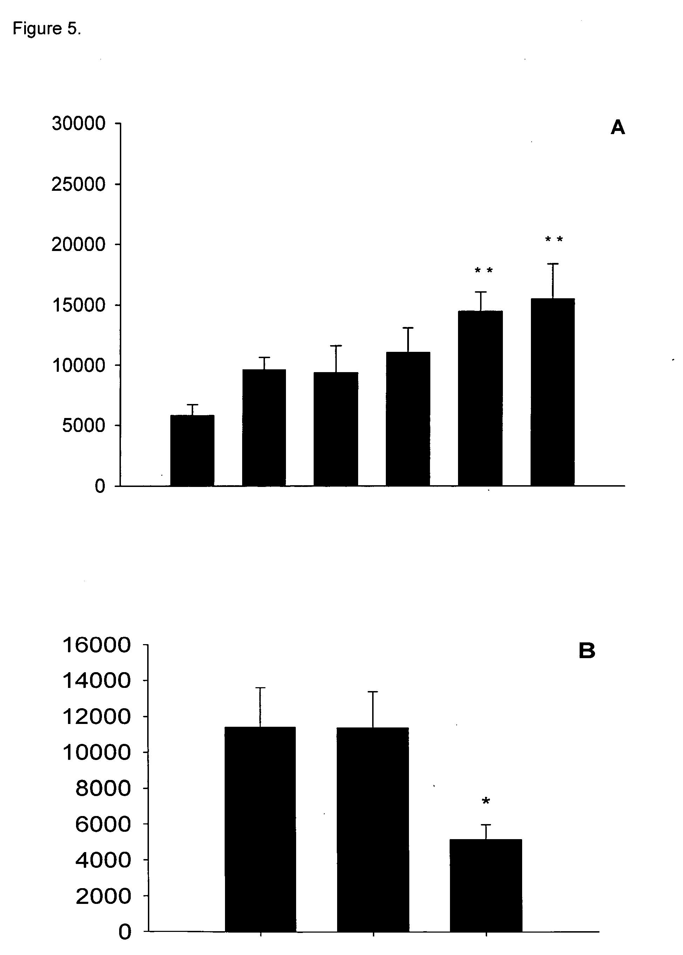

Figure 5a: Stimulation of cell growth of small lung cell carcinoma derived cell line NCI-H82 by binding of NNK to the α7 nicotinic acetylcholine receptor. (*) p<0.05 significant difference between NNK-treated cells and control group (Dunnett test). The bars correspond to the following NNK concentrations (from left to right): 0, 0.5 nM, 2.5 nM, 5 nM, 10 nM, 25 nM. The y-axis shows absolute cell numbers.
Figure 5b: P9D5 antibody (625nM) inhibits NNK-induced (25nM) proliferation in NCI-H82 Cells. (**) p<0.004 significant difference between control and P9D5 antibody. A calibration curve was used to convert ATP bioluminescence values into absolute cell numbers. Results are expressed as means ± SD. Control mAb 1 (P9E1 R4) and control mab 2 (mab13) are irrelevant antibodies. The bars correspond to (from left to right): control mAb 1, control mAb 2, P9D5 mAb. NNK. The y-axis shows absolute cell numbers.
Figure 6: Titration of anti-NNK sera with coated [NNK-C2]-OVA. open square: control mAb P9D5 (2mg/ml), solid circle: mouse serum sample 1, solid square: mouse serum sample 2, solid triangle: mouse serum sample 3. The x-axis shows the dilution factor of serum sample or control, the y-axis shows the O.D. (405 nm).

The examples illustrate the invention.

### Example 1: Synthesis of a NNK hapten

### Synthesis of 3-(2-pyridine-3-yl-[1,3]dioxolane-2-yl)-propylamine

### Preparation of 4-oxo-4-pyridine-3-yl-butyronitrile

A suspension of finely grounded sodium cyanide (490 mg, 10 mmol) in 50 ml dry N,N-dimethylformamide was vigorously stirred at 35°C for 15 min before undiluted 3-pyridinecarboxaldehyde (98%, 10.93 g, 100 mmol) was added dropwise over a period of 30 min. The dark brown solution was stirred for another 30 minutes, after which acrylonitrile (5.31 g, 100 mmol, 1 equiv.) was added over 1 h. The red-orange, viscous solution was mixed for 3 hours before glacial acetic acid (629 µl, 11 mmol) was added. After stirring of the solution for 5 more minutes, the solvent was partially removed under vacuum and the residue quenched with saturated aqueous NH₄Cl solution. The aqueous phase was extracted 4 times with CHCl₃, twice with ethyl acetate and mixed with CH₂Cl₂ overnight. The pooled organic extracts were dried (MgSO₄), the solvents evaporated *in vacuo,* and the residual oil purified by column chromatography (CC) on SiO₂ (ethyl acetate/petroleum ether: 4:1, 6:1, 1:0). Re-crystallization of the obtained orange colored solid from 2-propanol yielded 4-oxo-4-pyridine-3-yl-butyronitrile as yellow crystals (11.0 g, 69%). R_{f} 0.47 (ethyl acetate); ¹H-NMR (250 MHz, CDCl₃): δ 9.16 (1H, *d, J* = 2.0 Hz), 8.82 (1H, *dd, J* = 2.0 Hz), 8.24 (1H, *dt, J* = 2.0, 7.3 Hz), 7.49 (1H, *dd, J* = 4.4, 7.3 Hz), 3.40 (2H, *t, J =* 6.6 Hz, C*H*₂CH₂CN), 2.84 (2H, *t*, *J* = 6.6 Hz, CH₂C*H*₂CN) ppm; ¹³C-NMR (63 MHz, CDCl₃): δ 198.3, 154.2, 149.4, 135.4, 131.8, 124.0, 118.8, 34.5, 11.6 ppm; m/z (ES+) 161.1 ([M+H]⁺, 100%).

### Preparation of 3-(2-pyridine-3-yl-[1 ,3]dioxolane-2-yl)-propionitrile

A mixture of 4-oxo-4-pyridine-3-yl-butyronitrile (16 g, 0.1 mol), ethylene glycol (100 ml) and p-toluenesulfonic acid (20.9 g, 0.11 mol, 1.1 equiv.) in 150 ml dry toluene was refluxed for 2 days using a Dean-Stark apparatus. The oily reaction mixture was subsequently evaporated to dryness, the residue taken up in CHCl₃, and washed with saturated aqueous K₂CO₃ solution and ddH₂O. Purification of the crude product by CC (dichloromethane/methanol 50:1) yielded 3-(2-pyridine-3-yl-[1,3]dioxolane-2-yl)-propionitrile as a yellow oil (10.6 g, 52%). R_{f} 0.25 (92:8 dichloromethane/methanol); ¹H-NMR (250 MHz, CDCl₃): δ 8.7 (1H, *s*), 8.6 (1H, d, *J* = 4.7 Hz), 7.7 (1H, *dd, J* = 7.8, 1.8 Hz), 7.3 (1H, *dd, J* = 8.1, 5.1 Hz), 4.1 (2H, *m*), 3.8 (2H, *m*), 2.5 (2H, *t, J* = 7.5 Hz), 2.25 (2H, t, *J* = 7.5 Hz) ppm; ¹³C-NMR (63 MHz, CDCl₃): δ 149.9, 147.4, 136.7, 133.4, 123.3, 119.3, 107.3, 64.9, 35.6, 11.4 ppm; m/z (ES⁺) 205.1 ([M+H]⁺, 100%), 227.1 ([M+Na]⁺, 15%), 243.0 ([M+K]⁺, 20%).

### Preparation of 3-(2-pyridine-3-yl-[1,3]dioxolane-2-yl)-propylamine

3-(2-Pyridine-3-yl-[1,3]dioxolane-2-yl)-propionitrile (4.1 g, 20.0 mmol, 1 equiv.) was dissolved in 40 ml methanolic ammonia (methanol saturated with gaz. NH₃) and hydrogenated with H₂ in the presence of Raney-Ni (ca. 410 mg, 10 wt%) at 10 bar and 40°C for 3 days in a Parr hydrogenation apparatus. The catalyst was then removed by filtration on Celite and the filter-cake extensively washed with methanol to release the product trapped on the catalyst surface. The filtrate was evaporated in *vacuo* and the residue purified by CC on SiO₂ (dichloromethane/methanol/25% aqueous NH₃ solution 90:10:2) to yield 3-(2-pyridine-3-yl-[1,3]dioxolane-2-yl)-propylamine as a yellow oil (3.3 g, 79%). R_{f} 0.31 (90/10/2 dichloromethane/methanol/NH₄OH); ¹HNMR (250 MHz, CDCl₃): δ 8.7 (1H, *s*), 8.6 (1H, *d, J =* 5.5 Hz), 8.0 (1H, *d, J =* 7.9 Hz), 7.5 (1 H, dd, *J =* 7.8, 5.7 Hz), 4.1 (2H, *m*), 3.75 (2H, *m*), 3.6 (2H, t, *J* = 6 Hz), 2.3 (2H, *m*),2.0 (2H, *t, J* = 7.5 Hz), 1.60 (2H, q, *J* = 6.5 Hz) ppm; ¹³C-NMR (63 MHz, CDCl₃): δ 149.3, 147.7, 137.9, 133.6, 123.0, 109.2, 64.7, 42.1, 37.8, 27.7 ppm; m/z (ES⁺) 209.1 ([M+H]⁺, 100%).

### Synthesis of tert-butyl (2-iodo-ethyl)carbamate

### Preparation of tert-butyl (2-hydroxy-ethyl)carbamate

A solution of 2-aminoethanol in dry dichloromethane was treated with Et₃N (1.1 equiv.) at room temperature for 30 min before a solution of Boc₂O (1.1 equiv.) in dichloromethane was slowly added. The mixture was stirred at room temperature overnight and quenched with saturated aqueous NH₄Cl solution. The aqueous phase was extracted twice with dichloromethane and the combined organic extracts washed with brine, dried (MgSO₄) and concentrated *in vacuo* to give crude tert-butyl (2-hydroxy-ethyl)carbamate as a colorless oil (91-99 %). ¹H-NMR of the crude (N-Boc)-protected product showed that it was generally pure enough to be used in the next step without further purification. R_{f} 0.68 (20/1 dichloromethane/methanol); ¹H-NMR (250 MHz, CDCl₃): δ 4.96 (1H, *br*), 3.70 (2H, *dt*, *J =* 11.6, 5.8 Hz), 3.29 (2H, *dt*, *J* = 12.45, 6.2 Hz), 2.52 (*br*, OH), 1.44 (9H, *s*) ppm; ¹³C-NMR (63 MHz, CDCl₃): δ 159.0, 80.0, 62.6, 43.2, 28.3 ppm.

### Preparation of tert-butyl (2-iodo-ethyl)carbamate

Iodine (1.2 equiv.) was added portionwise to a solution of imidazole (1.2 equiv.) and PPh₃ (1.2 equiv.) in dichloromethane (50 ml) at 0°C. The resulting, deeply yellow colored suspension was warmed to room temperature before a solution of tert-butyl (2-hydroxy-ethyl)carbamate (1 equiv.) in dichloromethane was added. The reaction mixture was stirred at room temperature for 24 h, filtered over Celite and washed twice with 5 % aqueous Na₂S₂O₃ solution. The aqueous washing phases were extracted with dichloromethane and the org. phases dried (MgSO₄) and evaporated *in vacuo.* The residual yellow colored oil was subjected to purification by CC (Ether/ethyl acetate 1:1) and yielded the desired tert-butyl (2-iodo-ethyl)carbamate as a pale yellow oil (81%). R_{f} 0.51 (5/1 PET/ethyl acetate); ¹H-NMR (250 MHz, CDCl₃): δ 4.95 (1H, *br*), 3.50 (2H, *m*), 3.20 (2H, *m*), 1.47 (9H, *s*) ppm. ¹³C-NMR (63 MHz, CDCl₃): δ 155.8, 79.3, 43.0, 28.3, 5.9 ppm.

### N-alkylation of 3-(2-pyridine-3-yl-[1,3]dioxolane-2-yl)-propylamine with tert-butyl (2-iodo-ethyl)carbamate

### Preparation of {3-[3-(2-pyridine-3-yl-[1,3]dioxolane-2-yl)-propylamino]-ethyl}-carbamic acid tert-butyl ester

A solution of 3-(2-pyridine-3-yl-[1,3]dioxolane-2-yl)-propylamine (1 equiv.) in dry dimethylformamide (10 ml / mmol) was treated with Cs₂CO₃ (1.2 equiv., dried *in vacuo* prior to use) at room temperature for 30 min before a solution of tert-butyl (2-iodo-ethyl)carbamate (0.99-1.0 equiv.) in dimethylformamide (2 ml / mmol) was added dropwise. The pale yellow suspension was heated to 60°C, vigorously stirred overnight and the solvent evaporated under vacuum. The crude product was redissolved in dichloromethane and washed with saturated aqueous NH₄Cl solution and H₂O. The combined aqueous phases were extracted with dichloromethane (3x) and ethyl acetate (1x) and the collected org. layers washed with 5% aqueous Na₂S₂O₃ solution, dried (MgSO₄) and evaporated *in vacuo.* The residual oil was subjected to CC (dichloromethane/MeOH: 98:2, 95:5, 92:8, 90:10) to separate the mono-alkylation product 3-(2-pyridine-3-yl-[1,3]dioxolane-2-yl)-propylamine with tert-butyl (2-iodo-ethyl)carbamate (40%) from the over-alkylated material. R_{f} 0.29 (92/8 dichloromethane/methanol); ¹H-NMR (300 MHz, CDCl₃): δ 8.66 (1H, *s*), 8.53 (1H, *d*, *J* = 4.3 Hz), 7.73 (1H, d, *J* = 7.8 Hz), 7.27 (1H, *t*, *J =* 7.8 Hz), 6.26 (1H, *br*), 4.07 (2H, *t*, *J* = 6.8 Hz), 3.73 (2H, *t*, *J* = 6.8 Hz), 3.54 (2H, *m*), 3.09 (2H, *m*), 3.03 (2H, *m*), 1.99 (4H, *m*), 1.41 (9H, *s*) ppm; ¹³C-NMR (75 MHz, CDCl₃): δ 157.5, 150.4, 148.3, 138.4, 134.5, 124.0, 109.2, 80.5, 65.2, 48.7, 48.4, 43.5, 37.1, 28.5, 20.3 ppm; m/z (ES⁺) 252.1 ([M+H]⁺, 100%).

### Acid-catalyzed nitrosation of {3-[3-(2-pyridine-3-yl-[1,3]dioxolane-2-yl)-propylamino]-ethyl}-carbamic acid tert-butyl ester

### Preparation of {3-[3-(2-Pyridine-3-yl-[1,3]dioxolane-2-yl)-propyl(nitrosamino)]-ethyl}-carbamic acid tert-butyl ester

{3-[3-(2-Pyridine-3-yl-[1,3]dioxolane-2-yl)-propylamino]-ethyl}-carbamic acid tert-butyl ester (1 equiv.) was suspended in ddH₂O (2.5 ml/mmol), the oily droplets dissolved through addition of 1 N aqueous HCl solution and the pH of the resulting homogenous layer adjusted to 5-6 with 25% aqueous NaOH solution After cooling the solution to 0°C, an aqueous solution of NaNO₂ (2 equiv.) was added dropwise over 20 min.
The reaction was allowed to proceed at room temperature overnight, then diluted with dichloromethane and alkalinized with saturated aqueous NaHCO₃. The basic aqueous layer was extracted with dichloromethane (3x) and ethyl acetate (1x), the combined org. layers dried (MgSO₄), evaporated and purified by CC on SiO₂ (dichloromethane/methanol: 98:2, 95:5). {3-[3-(2-Pyridine-3-yl-[1,3]dioxolane-2-yl)-propyl(nitrosamino)]-ethyl}- carbamic acid tert-butyl ester was obtained as a yellow oil (53-75%). R_{f} 0.47 (95/5 dichloromethane/methanol); ¹H-NMR (250 MHz, CDCl₃): δ 8.70 (1H, *s*), 8.59 (1H, d, *J* = 4.5 Hz), 7.79 (1H, d, *J* = 7.8 Hz), 7.32 (1H, *t*, *J* = 5.1 Hz), 4.79 (1H, *br*), 4.12 (2H, *m*), 4.06 (2H, *m*), 3.78 (2H, *m*), 3.36 (2H, *m*), 3.19 (2H, *m*), 1.92 (2H, t, *J* = 7.8 Hz), 1.59 (2H, *m*), 1.44 (9H, *s*) ppm; ¹³C-NMR (63 MHz, CDCl₃): δ 155.8, 149.5, 147.5, 137.7, 133.5, 123.3, 108.7, 82.4, 64.7, 52.1, 51.8, 43.8, 37.2, 27.7, 22.4 ppm; m/z (ES⁺) 381.0 ([M+H]⁺, 100%).

### Cleavage of protection groups

### Preparation of 4-[2-Amino-ethyl(nitrosamino)]-1-pyridine-3-yl-butan-1-one

{3-[3-(2-Pyridine-3-yl-[1,3]dioxolane-2-yl)-propyl(nitrosamino)]-ethyl}- carbamic acid tert-butyl ester (1 equiv.) was dissolved in ethyl acetate (1 ml/mmol) and treated with a 5-fold excess of conc. aqueous HCl (10N, 5 equiv.) at reflux overnight. The cloudy reaction mixture was then cautiously alkalinized to pH 14 with 25% aqueous NaOH solution, and the aqueous phase vigorously extracted with dichloromethane (3x) and ethyl acetate (2x). The combined organic phases were washed with brine, dried (MgSO₄) and evaporated *in vacuo.* The residual oil was purified by CC (dichloromethane/methanol/25% aqueous NH₃ solution 90:10:3) to give 4-[2-Aminoethyl(nitrosamino)]-1-pyridine-3-yl-butan-1-one as a yellow oil (40-45%), which was stored at -20°C under argon. The free amine 4-[2-Amino-ethyl(nitrosamino)]-1-pyridine-3-yl-butan-1-one was then converted into a stable hydrochloride salt by HCl/methanol-treatment for 2 h at room temperature and subsequent drying under vacuum. R_{f} 0.27 (90/10/3 dichloromethane/methanol/NH₄OH); ¹H-NMR (200 MHz, D₂O): δ 9.22 (1H, *s*), 8.97 (1H, *d, J =* 6.8 Hz), 8.88 (1H, *d, J =* 4.5 Hz), 8.10 (1H, *dd, J* = 6.8,4.5 Hz), 3.34 (1H, *q, J* = 2.6 Hz ), 3.26 (2H, *t*, *J* = 5 Hz), 3.14 (2H, q, *J* = 5 Hz), 3.03 (2H, *t*, *J* = 6.2 Hz), 2.1 (2H, q, *J* = 5 Hz) ppm; ¹³C-NMR (50 MHz, D₂O): δ 197.0, 145.2, 144.9, 142.06, 134.6, 127.7, 48.9, 47.2, 38.7, 35.6, 20.6 ppm.

### Example 2: Conjugation of 4-[2-Amino-ethyl(nitrosamino)]-1-pyridine-3-yl-butane-1-one to proteins

A two-step zero-length cross-linking procedure using active esters was adopted for conjugating 4-[2-amino-ethyl(nitrosamino)]-1-pyridine-3-yl-butane-1-one (NNK-C2) to ovalbumine (OVA, 45.000 g/mol), and diphtheria toxoid (DT, 63.000 g/mol), which is commercially available. OVA (10 mg/mL) was dissolved in 100 mM TrisHCl buffer pH 7.5. Crude DT was purified by buffer exchange using a 10 kDa cut-off amicon (Millipore, Marlborough, USA) in 100 mM TrisHCl buffer pH 7.5 and the solution adjusted to 10 mg/mL concentration. 1mL of both protein solutions were then reduced by treatment with 1mL of dithio-1,4-threitol (2 mg/mL) in 100 mM TrisHCl buffer pH 7.5 and 7 mL of ddH2O at 50°C for 15 minutes. The reduced proteins were subsequently alkylated without further purification by addition of 1 mL of iodoacetamide (5 mg/mL) in 100 mM TrisHCl buffer pH 7.5 After stirring the mixture for 15 minutes at room temperature, the reduced and alkylated protein solutions were purified by buffer exchange in 100 mM MES buffer pH 6 using a 10 kDa cut-off amicon cell. The solution titer were determined by standard protein quantification assays kit (BCUptima, Amersham Biosciences, San Francisco, USA). and the concentration adjusted to 10 mg/mL by dilution. In a second step, 100 µL of the reduced and alkylated proteins solutions (10 mg/mL) were activated for 15 minutes at room temperature with a mixture of 200µL of sulfo-N-hydroxy-succinimide (8 mg/mL, Pierce Rockford, USA; in 100 mM MES buffer pH 6), 200µL of 1-ethyl-3-(3-dimethylaminopropyl) carbodiimide hydrochloride (2.75 mg/mL in 100 mM MES buffer pH 6) and 400 µL of ddH2O. After activation the excess reagents were quenched at room temperature for 10 minutes with 100 µL of β-mercaptoethanol (20 mg/mL in 100 mM MES buffer pH 6). Finally, 1 mL of NNK-C2 (20 mg/mL) in PBS (0.1 M sodium phosphate, 0.15M NaCl at pH 7.5) was added to the activated protein solutions and the mixtures left to react overnight in the dark. The bio-conjugate solutions were purified by buffer exchange with 50mM ammonium bicarbonate buffer pH 7.8 using a 10 kDa cut-off amicon cell and quantified through standard protein quantification kit (BC-Uptima, Amersham Biosciences, San Francisco, USA).
Positive ion MALDI-TOF mass spectrometry was performed on the NNK-C2 conjugates using a Bruker Daltonics ULTRAFLEX TOF/TOF equipped with a 337nm, 50 Hz N₂ laser of 100µj. The crystal matrix, 3,5-dimethoxy-4-hydroxycinnamic acid (sinapinic acid) from Bruker Daltonics (Leipzig, Germany) was prepared at a concentration of 10 mg/mL in trifluoroacetic acid 0.2%/acetonitrile 75:25. Protein samples were 1 mg/mL in 50mM ammonium bicarbonate buffer pH 7.8. 0.5µl Sample and 1µl matrix solutions were mixed directly on the stainless steel probe and allowed to dry (10 minutes) at room temperature. Spectra were recorded in the linear mode and the resulting data were analyzed using the software supplied by Bruker Daltonics. The instrument was calibrated using a 10 mg/mL BSA solution in ddH₂O.

The small synthetic hapten NNK-C2 was coupled to the large carrier proteins OVA and DT using a standard active ester procedure (Z. Grabarek et al., Anal. Biochem. (1990) 185, 131) as described above. Before conjugation the native proteins were reduced and alkylated with iodo-acetic acid. In all cases a minimum of a 120-fold molar excess of hapten over the protein carboxylic reactive groups was used. After the coupling reactions, the protein conjugates were purified by dialysis. Molecular weights of protein conjugates were determined by MALDI-TOF mass spectrometry and the average unit of hapten per carrier estimated by mass difference. The observed typical mass difference between free DT (61350 Da) and conjugated DT ([NNK-C2]-DT, 62650 Da) of 1.3 kDa corresponds to an average number of approximately 5.5 NNK-C2 molecules (235.1 Da) per molecule of carrier. Analysis of OVA conjugate gave very similar estimated numbers of hapten per protein.

The above conjugates will be referred to as [NNK-C2]-DT and [NNK-C2]-OVA, respectively.

### Example 3: Monoclonal antibody production

### Immunization

Groups of five 8-10 weeks old pathogen-free BALB/c mice were primed intraperitoneally (i.p.) with 25 µg of [NNK-C2]-DT using complete Freund's adjuvant (v/v) (Sigma). Mice were boosted by gastric intubation on day 14, 16, 28, 30, 42 and 44 with the same dose of antigen in the presence of 5 µg cholera toxin (Sigma). Sera were drawn on day 0, 14, 28, 42, 55 and 84 and anti-NNK-C2 antibody titer was measured by direct ELISA. Three days prior to the hybridoma fusion, a final boost was performed i.p. with incomplete Freund's adjuvant (v/v) (Sigma) to two mice with the highest NNK-C2 specific antibody titers.

### Hybridoma production

Hybridomas were produced using ClonaCell^{™}-HY complete kit (StemCell Technologies). Briefly, from immunized mice spleen, 108 splenocytes were washed and incubated in PEG-containing medium with 2.107 parental myeloma cells, SP2/0 for 5 min at 37°C. After a 24 hours expansion, cells were mixed with a methylcellulose-based HAT (hypoxanthine, aminopterin and thymine) selective medium for hybridomas, the mixture was plated on dishes and incubated at 37°C, allowing fused cells to growth for 14 days.
Hybridoma colonies were picked and transferred into 96-well plates with growth medium. After 4 days of growth at 37°C, each supernatant was tested for the presence of specific [NNK-C2]-antibodies by direct ELISA. Positive clones were further adapted to RPMI-1640 medium supplemented with HyClone HyQ PF-Mab (Perbio Sciences), a protein-free nutrient supplement for mAb production.

### Induction of NNK-specific antibodies

Specific antibodies were monitored after each injection of [NNK-C2]-DT. Antibodies against the homologous conjugate appeared within two weeks after the first injection. Significant levels of NNK-C2 specific antibodies were detected with [NNK-C2]-OVA after the second injection and increased with each boost. No specific IgA antibodies were detected despite the partially oral route. NNK-specific antibodies were monitored by competition ELISA by adding increasing concentrations of NNK as competitors (0-256µM) (Figure 1) to measure their capacity to inhibit the binding of serum antibodies to coated [NNK-C2]-OVA. The IC₅₀ of NNK and NNAL was 26 µM and 80 µM respectively, while the IC₅₀ of the hapten NNK-C2 was 16 to 50 times lower (Figure 1a). Also 4-Hydroxy-1-(3-pyridyl)-1-butanone (HPB) (80 µM) and 1-(3-pyridyl)-1-butanediol (PB) (230 µM) were recognized by mouse sera, while essentially no specific reactivity was detected against the detoxified N-oxide NNK metabolites 4-(methylnitrosamino)-1-(3-pyridyl-N-oxide)-1-butanone (NNK-N-Ox) and 4-(methylnitrosamino)-1-(3-pyridyl-N-oxide)-1-butanol (NNAL-N-Ox).

### Example 4: Characterization of antibodies

### Indirect ELISA

384-well microtiter plates (Microlon, Greiner) were coated overnight with 20µl of 0.125 µM [NNK-C2]-OVA in 0.1 M bicarbonate buffer (pH 9.6). After washing, free binding sites were saturated with 1% BSA in Tris-buffered (15mM) saline (TBS, pH 7.4). 20µl of diluted mouse serum in TBS containing 0.1% Tween-20 or non-diluted supernatants were incubated for 90 min at room temperature. Alkaline phosphatase-conjugated goat anti-mouse mAb (1:750; Southern Biotechnology Associates) and a substrate solution of 0.05% *p*-nitrophenyl phosphate in 1 mM 2-amino-2-methyl-1-propanol (Sigma) and 0.1 mM MgCl₂ (pH 10.2) were used to detect antibody binding. Optical density (O.D.) was measured after 60 min at 405 nm. Net optical density (OD) was calculated by subtraction of the relevant background as described in each figure.

### Competition ELISA

In all competition experiments, optimal antigen coating conditions and serum dilutions were determined by direct ELISA. 384-well plates were coated with the minimal amount of antigen required for maximal signal. Sera or mAb supernatants were diluted to obtain 70% binding and a signal of 1.0 to 1.5 O.D. at 0% competition (highest signal). The competitors (NNK and its metabolites (NNAL, NNK-N-ox, NNAL-N-ox, keto acid (1-(3-pyridyl)-1-butanone-4-carboxylic acid), PBCA (1-(3-pyridyl)-1-butanol-4-carboxylic acid), PB (1-(3-pyridyl)-1-butanediol) and HPB (4-hydroxy-1-(3-pyridyl)-1-butanone)), from Toronto Research Chemicals Inc., Canada and Acros Organics, Geel, Belgium) were resuspended in 100% dimethylsulfoxide and stock solutions of 100 µM were frozen at -80°C. Competitors were mixed 1:1 with sera or mAb supernatants to final concentrations of 0 to 512 µM for mAbs or 0 to 1024 µM for serum. Zero (highest signal) and 100% competition (background signal) were determined using no or NNK-C2 as competitor. The difference between these two values corresponds to the dynamic range of the assay. For each competitor concentration, the 50% of inhibitory concentration, IC₅₀ was calculated. Experiments were independently repeated three times and data were analyzed used SigmaStat software. Net OD was calculated by subtraction of the relevant background as described in each figure.

### Affinity measurements by surface plasmon resonance.

BIACORE 3000 instrument, CM5 sensor chip, HBS-EP buffer [10 mmol/L HEPES, 0.15 mol/L NaCl, 3 mmol/L EDTA, and 0.005% surfactant P20 (pH 7.4)], amine coupling kit (N-hydroxysuccinimide; N-ethyl-N'-dimethylaminopropylcarbodiimide), and ethanolamine were obtained from GE Healthcare (Diegem, Belgium). [NNK-C2]-DT and DT were immobilized on CM5 sensor chips according to standard amino coupling procedure. Briefly, the carboxymethylated dextran-coated surface was activated by a 7-minute injection of a solution containing 200 mmol/L 1-ethyl-3-(3-dimethylaminopropyl) carbodiimide hydrochloride and 50 mmol/L N-hydroxysuccinimide; then, [NNK-C2]-DT or DT (10 µg/mL in 10 mmol/L sodium acetate pH 4.5) were injected. A continuous flow of HBS-EP at 5 µL/min was maintained and capping of unreacted sites was achieved by injecting 1 mol/L ethanolamine-HCl (pH 8.5). Final immobilization responses of 500 resonance units. Sensorgrams for kinetic measurements were generated by injection of mAbs (at nine concentrations ranging from 0 to 100 nM) in HBS-EP at a flow rate of 30 µL/min. The association and dissociation times were 120 and 900 seconds, respectively. The chip was regenerated by injection of consecutive pulses of 10 mM glycine pH 1.5 for 30 s and 10 mM glycine pH 2 for 60 s until the difference between the baselines before and after injection was <10 resonance units. Biosensor data were prepared, modeled, and fitted by means of the BlAevaluation 4.1software. Data from the reference surface were subtracted to remove the effects of non specific binding in order to calculate the specific binding. The presence of mass transfer phenomena were excluded by performing the control assays as recommended by BIACORE. Evaluation of the data was done using a bivalent-analyte model with simultaneous determination of association and dissociation constants. The quality of the fitted data was evaluated by comparison between calculated and experimental curves (residual values) and by the magnitude of the X² parameter.

### Results:

The spleen of a mouse with high levels of specific antibodies to NNK-C2 and NNK was used for the production of mAbs. We described here two IgG1 mAbs (P9D5 and P7H3) that reacted strongly with [NNK-C2]-OVA.

The affinity of P9D5 and P7H3 toward the hapten [NNK-C2] was estimated by surface plasmon resonance (BiaCore) following the bivalent model evaluation. The following values were obtained in the above described experiment: Kd of P9D5 was 45.8 nM (ka1 = 1.11e5 ±202; kd1 = 5.09e-3 ±4.25e-5; X² = 7.07); Kd of P7H3 was 37.6 nM (ka1 = 1.99e5± 334; kd1 = 7.49e-3 ± 8.38e-5; X² = 5.68). ka is the association rate constant, kd is the dissociation rate constants. The affinity dissociation constant is KD= kd/ka.

The reactivity of P9D5 and P7H3 was assessed by competition ELISA using NNK metabolites and other derivatives as competitors. Binding was inhibited by NNK-C2 (range of IC₅₀: 4.4-12 µM), NNAL (range of IC₅₀: 29-93 µM) and by NNK (range of IC₅₀: 80-160 µM).
Furthermore, the binding of P9D5 was strongly inhibited by HPB (IC₅₀: 50 µM), PB (IC₅₀: 72 µM) and ENAT (ethylnicotinate) (IC₅₀: 49 µM). A moderate inhibition of P9D5 binding was observed with keto acid and with PBCA. Weak or no competition was measured with the other metabolites and derivatives. P7H3 showed a similar reactivity, its binding was strongly inhibited by ENAT (IC₅₀: 80 µM), moderately inhibited by HPB, PB, keto acid and PBCA and weakly or not inhibited by the other metabolites and derivatives.
P9D5 monoclonal antibody, an IgG1 isotype, was characterized by competition ELISA using different NNK metabolites. Figure 2A shows that NNK-C2, NNK and NNAL bind to the antibody albeit with different affinities. The strongest binder was the unconjugated NNK-C2, the hapten used for coupling and immunization (IC₅₀: 3.7 µM). The IC₅₀ for NNK was considerably lower (63 µM). Interestingly, binding of P9D5 to the metabolite NNAL (IC₅₀: 20 µM) was 3 times stronger than to NNK. Figure 2B shows that HPB (IC₅₀: 50 µM) and PB (IC₅₀: 72 µM), two major metabolites of the P₄₅₀-dependent pathway, bind with similar affinity as NNK. In contrast, the detoxified N-oxide metabolites of both NNK and NNAL were not recognized by the antibody.

The above and further experiments show that in contrast to the antibodies induced against the pyridyl-oxobutyl moiety (Talbot, B. et al., Arch. Toxicol. (1990) 64, 360-364) and in contrast to the antibodies described in US-A-2005/0043515, the antibodies described in this example specifically recognize NNK and even better NNAL. The competition capacity of the pyridyl ring (IC₅₀ = 400 µM or 320 µM for 3-picoline) corresponds to 25%-40% of the competition capacity of NNK (IC₅₀ = 80 µM or 160 µM) for P9D5 or P7H3 calculated from the OD using the dynamic range of the assay. The N-oxidation of the pyridyl ring (e.g. NAD-N-Ox (nicotinamide-N-oxide), NIA-N-Ox (nicotinic acid-N-oxide), NNK-N-Ox and NNAL-N-Ox) totally obliterates binding of the antibodies described herein. This shows that the pyridyl ring is an important part of the epitope, and the antibody paratope cannot accommodate the bulkier and charged groups of the species having an oxidized pyridyl moiety. The side chain in position 3 of the pyridine ring improves the recognition as exemplified by the efficient binding of ethyl nicotinate (IC₅₀ = 49-80 µM). P7H3 and P9D5 show differential sensitivity to functional groups on the side chain. P7H3 requires the presence of the nitroso group which is absent in HPB, PB, ketoacid and PBCA. In contrast, P9D5 is less sensitive to modification at the nitroso group, with the IC₅₀ of HPB and PB being similar to the one of NNK, but this antibody is sensitive to the acid moiety in ketoacid and PBCA with a significant increase of the IC₅₀. The carbonyl reduction of NNK to NNAL improves the binding by 2-3 fold, although this is not the case for the acid end-point metabolites thereof.

### Example 5: Specific Apical Antibody Inhibits the Absorptive Transport of [5-³H]NNK Across Calu-3 Monolayers

Calu-3 cells were maintained in EMEM (Biowhittaker, Verviers, BE) supplemented with 10% heat-inactivated fetal bovine serum, 1% MEM non-essential amino acids, 1 mM sodium pyruvate, 2 mM glutamine, 100 IU/ml penicillin and 100 µg/ml streptomycin ("Calu-3 medium") in a humidified incubator (5% CO₂, 37 °C). Every 3 to 4 days when they reached 80% confluency, monolayers were subcultured by treatment with 0.05% trypsin and 0.02% ethylene diamine tetra acetic acid (EDTA).

### Transport Experiments

Passages 131-142 of Calu-3 were seeded at a density of 50.000 cells/cm² on Clear Transwell inserts (0.4 µm pore diameter, 6.5 mm diameter; Corning Costar Inc., NY). Monolayers were allowed to grow for 1 week; confluence was reached within 2 days. The culture medium was changed every other day. Before the experiments, cells were provided with new Calu-3 medium in both the apical and basolateral compartment and kept for 1 to 2 h under standard tissue culture conditions. The integrity of the monolayers was checked before and after each experiment by measuring the Transepithelial Electrical Resistance (TEER) with an epithelial End Ohm Voltohmmeter (EVOM; World Precision Instruments, Aston, UK). Only monolayers with TEER-values higher than 550 Ω·cm² were used. The inclusion of P9D5 antibody or irrelevant antibody did not affect integrity of the Calu-3 monolayers. As an additional confirmation of integrity, sodium fluorescein (20 µg/ml) diffusion across the Calu-3 monolayers was assessed at the end of each experiment. Concentrations of sodium fluorescein samples were determined in black 96-well plates (Greiner, Bio-one, Wemmel, BE) using a GENios Plus fluorescence plate reader (Tecan, NL) at excitation and emission wavelengths of 485 and 530 nm, respectively. Typical sodium fluorescein fluxes were lower than 0.5% h-1.
Transport was initiated by adding 500 µl of fresh Calu-3 medium to the basolateral compartment and 150 µl of [5-³H]NNK (Chemsyn Laboratories, Lenexa, KS, USA, 2.5 Ci/mmol) in Calu-3 medium to the apical compartment. The NNK-specific antibody (P9D5) was co-incubated in either the apical or basolateral compartment. Controls received no antibody or irrelevant antibody P9E1 R4. Samples of 500 µl were drawn from the receiver compartment at 0.25, 0.5, 0.75, 1, 2 and 3 hours. To maintain sink-conditions, the Transwell filters were placed in fresh wells containing 500 µl of pre-warmed Calu-3 medium after each sampling. Medium samples (500 µl) were mixed with 2 ml of scintillation cocktail (Optiphase Hisafe2, Perkin Elmer, BE) and assessed by scintillation counting (1209 RackBeta, LKB, Wallac, BE). Each experiment was performed in triplicates and repeated at least twice. Toxic effects of [5-³H]NNK or antibody were excluded by TEER measurements before and after each transport experiment. Results are expressed as concentration of NNK in both the acceptor and donor compartment at the end of each incubation.

### Uptake Experiments

For uptake experiments, Calu-3 cells were plated at a density of 50.000 cells/cm² on collagen type I coated 96-well plates (Greiner Bio-one, Wemmel, BE). Experiments were conducted using monolayers 7 days after seeding. Monolayers were rinsed with fresh Calu-3 medium before adding NNK (80 nM, 250 µl, 20 pmol) in Calu-3 medium in the presence of a 65-fold molar excess of either a NNK-specific antibody (P9D5, 5.3 µM, 1320 pmol) or the irrelevant control antibody (P9E1 R4, 5.3 µM, 1320 pmol). Controls consisting of [5-³H]NNK added to Calu-3 medium without cells were included. After 24 h, 48 h, and 72 h supernatants were harvested and frozen at -20°C until further processing for HPLC analysis. Experiments were performed in triplicates and repeated at least twice.

### HPLC Analysis

An aliquot of the cell culture medium (250 µl) was transferred to Eppendorf vials, mixed with 10 µl of mixture of the following unlabeled UV-absorbing reference metabolites (Carmella S. G. et al., Anal. Biochem. 1985;145: 239-244): PBCA, keto acid, NNK-N-Oxide, NNAL-N-Oxide, PB, HPB, NNAL and NNK (all from Toronto Research Chemical Inc., ON, Canada). Proteins were precipitated with 150 µl of 0.3 N barium hydroxide (Sigma Aldrich, Bornem, BE) and 300 µl of 25% zinc sulfate (Merck, Darmstadt, GE). Each sample was vortexed, centrifuged (10,000 g, 15 min) and supernatants were transferred to new Eppendorf vials. A sample of 600 µl of clear supernatant was first diluted with 100 µl of 1 M triethyl ammonium acetate pH 7.0 (Sigma Aldrich, Bornem, BE) and the volume was adjusted with HPLC-grade water to a final volume of 1.05 ml.
Analysis of NNK metabolites in medium was performed according to a modified procedure developed by Carmella and Hecht (Carmella S. G. et al., Anal. Biochem. 1985;145: 239-244; Schrader et al., Drug Metab. Dispos. 2000;28:180-185). HPLC analysis was performed on the Amersham Pharmacia Biotech Akta Explorer 10S equipped with a P-900 Pump, a UV-900 detector and a Frac-950 fraction collector.

All solvents were of HPLC grade (Merck, Darmstadt, GE) and degassed under vacuum prior to use and continuously degassed under vacuum throughout the analyses. Samples (1 ml) were analyzed on a 4 x 250 mm LiChrospher 60 RP-SelectB 5-µm column fitted with a 4 x 4 mm precolumn (Merck, GE) by elution with a gradient that was linear from 100% buffer A to 70% A / 30% B in 25 min and linear to 40% A / 60% B in 5 min. Buffer A consisted of 20 mM Tris-HCl buffer (pH 7.2) and buffer B consisted of 20 mM Tris-HCl buffer (pH 7.2) containing 50% of acetonitrile. The flow rate was 0.7 ml/min. Radioactive metabolites in the elute were identified by cochromatography with unlabeled reference compounds detected by UV absorption at 254 nm. 500 µl fractions were collected and radioactivity was measured by liquid scintillation counting in Optiphase Hisafe2 (Perkin Elmer, BE). The recovery of total radioactivity during HPLC analysis was more than 90%. Peaks with counts less than twice the background were considered insignificant.

Calu-3 cells were used as a model of lung epithelial cells because they form tight monolayers and are an accepted model to examine pulmonary transport of low molecular weight substances and xenobiotics (Foster K. A. et al., Int. J. Pharm. 2000;208:1-11). In a first set of experiments, we investigated in the Transwell system how an apical antibody against NNK would modulate the absorptive transport of NNK. Different apical conditions were used (Fig. 3), while the basolateral condition was kept constant (Calu-3 medium). 130 nM [5-³H]NNK (20 pmol) in Calu-3 medium was added to the apical side together with increasing amounts of P9D5 antibody (1650 - 6600 nM, 250-1000 pmol) or the irrelevant control antibody P9E1R4 (6600 nM, 1000 pmol). Figure 3A shows that the transepithelial transport of [5-³H]NNK decreased with increasing concentrations of the specific antibody. A 50-fold molar excess of P9D5 antibody (6600 nM, 1000 pmol) added to 20 pmol [5-³H]NNK in the apical compartment decreased the transepithelial transport by more than 50% compared to control antibody (Figure 3A) or no antibody (data not shown). Figure 3B shows that the co-addition of [5-³H]NNK with the 50-fold molar excess of P9D5 antibody resulted after 3h in a recovery of about 60% of the amount of [5-³H]NNK initially administered, corresponding to apical concentrations that were about 30% higher when compared to no antibody or a 50-fold molar excess of control antibody. These results suggest that apical or luminal antibodies at the respiratory tract may modulate the uptake of NNK by lung epithelium and thus, potentially decrease local metabolic activation as well as systemic uptake.

### Example 6: Basolateral Antibody does not Enhance the Absorptive Transport of [5-³H]NNK Across Calu-3 Monolayers

The experiments were carried out according to the procedures described in example 5.

In these experiments, we investigated how basolateral antibodies would modulate absorptive transport of NNK. The apical condition was kept constant (130 nM [5-³H]NNK, 20 pmol), while increasing amounts of the NNK antibody P9D5 (1650 - 3300 nM, 825-1650 pmol) were added to the basolateral compartment. Control wells contained no antibody or irrelevant antibody (3300 nM, 1650 pmol). It was shown that both the transepithelial transport and the disappearance of [5-³H]NNK from the apical compartment were not influenced by basolateral antibody compared to controls with no antibody or irrelevant control antibody.

### Example 7: Specific Antibody Modulates the Metabolism of [5-³H]NNK in Calu-3 Monolayers

The experiments were carried out according to the procedures described in example 5.

In the following uptake experiments, the dose-dependent effect of antibody on both the generation of NNK-metabolites and the disappearance of NNK was investigated using Calu-3 cells cultivated in collagen-coated 96-well-plates. Cells were incubated for 24-72 h with 80 nM [5-³H]NNK (20 pmol) in the presence of NNK P9D5 antibodies (2700 - 5300 nM, 660-1320 pmol). Figure 4A shows that the co-addition of [5-³H]NNK (80 nM, 20 pmol) with a 65-fold molar excess of P9D5 antibody (5300 nM, 1320 pmol) resulted after 72 h in a recovery of about 65% of the initial input [5-³H]NNK, corresponding to concentrations that were more than 2-fold higher compared to a similar molar excess of control antibody (Fig. 4A) or no antibody (data not shown). Recovery of [5-³H]NNK decreased rapidly when a 2-fold lower antibody concentration was used. Figure 4B shows that binding of [5-³H]NNK by P9D5 antibody resulted in a concomitant dose-dependent decrease of NNAL formation. Under the conditions of the assay, the only metabolite detectable in the supernatants at all time points was NNAL, irrespective of the presence or absence of specific or control antibody. In the presence of a 65-fold molar excess of P9D5 antibody (5300 nM, 1320 pmol), NNAL formation was reduced by 46% and 54% compared to control antibody at 48h and 72h, respectively. In the presence of a 33-fold molar excess of P9D5 antibody (2700 nM, 660 pmol), NNAL formation was reduced by 27 % and 20 % compared to controls at 48 h and 72 h, respectively. These experiments show that extracellular sequestration of NNK by specific antibody may modulate its metabolic conversion to NNAL, and thus may be expected to reduce DNA adduct formation in lung cells.

### Example 8: Specific Antibody Prevents NNK-induced Proliferation in NCI-H82 Cells

NCI-H82 cells were obtained from European Collection of Cell Cultures and maintained in RPMI 1640 with 2 mM L-glutamine (Biowhittaker, Verviers, BE) with the above supplements ("NCI-H82 medium"). Every 2 or 3 days cell medium was renewed. Cells were subcultured once a week. All cultures were mycoplasma-free.

For proliferation experiments, NCI-H82 cells were seeded at a concentration of about 8 x 10³ cells/well in 200 µl of NCI-H82 medium on collagen type I coated 96-well plates (Greiner Bio-one, Wemmel, BE) and incubated at 37°C and 10% CO₂. Proliferation assays were performed at 10% CO₂ since CO₂ seems to act as a potent messenger molecule for SCLC cells, enhancing the susceptibility of this cancer cell to a host of autocrine and paracrine growth-stimulating signals. After 18h, medium was replaced by fresh NCI-H82 medium containing only 0.15% heat-inactivated fetal bovine serum ("low serum medium") 6h prior NNK treatment. Stock solutions of NNK were prepared in dimethyl sulfoxide. 100 µl of NNK in low serum medium were added to the corresponding wells to obtain final concentrations of 0.5 nM to 25 nM. Final concentration of dimethyl sulfoxide was less than 0.1% in all cultures. Cell proliferation was assessed 24h after treatment using the ViaLightTM HS kit according to the manufacturer's instructions (Cambrex Bioscience, Verviers, BE). Bioluminescence of ATP present in metabolically active cells, was measured on a GENios Plus fluorescence plate reader (Tecan, Mechelen, BE). Experiments were performed in sextuplicates and repeated at least twice. Differences in the aggregation state of NCI-H82 cells lead to 3 to 4-fold differences in absolute numbers of cells between independent experiments.

Binding of NNK to the α7-nicotinic acetylcholine receptor stimulated the proliferation of the small cell lung carcinoma cell line NCI-H82 in dose dependent and significant fashion, as shown by proliferation assay. In a typical experiment as the one of Figure 5 NNK (25 nM) increased the cell number by 2 to 3 fold in comparison to unstimulated control cells (p<0.05). This effect of NNK was inhibited in the presence of a 25-fold molar excess (625 nM) of NNK-specific monoclonal antibody P9D5 (Figure 5, insert). In comparison to similar concentrations of irrelevant antibodies (P9E1 R4, mab13) the specific antibody reduced proliferation by more than 50% (p<0.016 and p<0.004; Mann-Whitney Rank Sum test), corresponding essentially to the proliferation rate of the unstimulated control.

## Claims

1. A compound of formula (I) 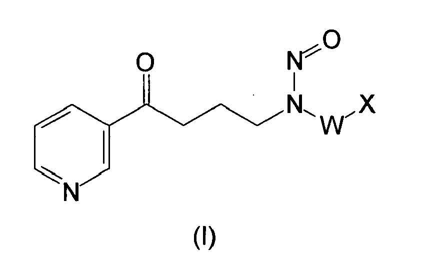 wherein
X is a functional group which allows coupling of the compound of formula (I) to an immunogenic carrier;
W is C₁₋₁₂ alkylene, wherein one or more CH₂ are optionally replaced by O, provided that each O is separated from the N and the X, to which W is attached, and from the other O's, if present, by at least two CH₂.

2. The compound of claim 1, wherein W is C₂₋₆ alkylene.

3. The compound of claim 1 or 2, wherein X is COOH, NH₂ or SH.

4. A method for the preparation of the compound of claim 1, comprising:
nitrosating a compound of formula (II) 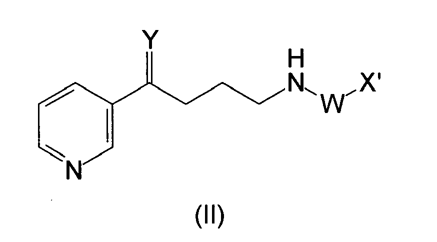
wherein
X' is a protected functional group, wherein the functional group, after removal of the protecting group, allows coupling of the compound of formula (I) to an immunogenic carrier;
=Y is a protected carbonyl group;
W is a C₁₋₁₂ alkylene, wherein one or more CH₂ are optionally replaced by O, provided that each O is separated from the N and the X', to which W is attached, and from the other O's, if present, by at least two CH₂; and removing the protecting groups to convert =Y into a carbonyl group and to convert X' into X, wherein X is a functional group which allows coupling of the compound of formula (I) to an immunogenic carrier.

5. The method of claim 4, wherein the nitrosating step is preceded by a step of reacting a compound of formula (III) 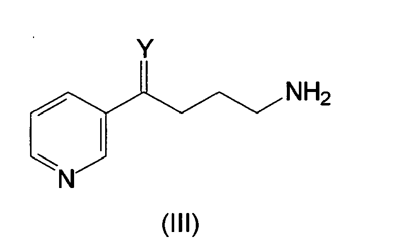 wherein =Y is as defined in claim 4;
with a compound of formula (IV)
Z-W-X'
(IV)
wherein
X' is as defined in claim 4;
W is as defined in claim 4; and
Z is a leaving group.

6. The process of claim 5, wherein Z is I.

7. The process of claim 5 or 6, wherein X' is COOR', NHR' or SR', wherein R' is a protecting group.

8. A conjugate obtainable by coupling a compound of any one of claims 1 to 3 to an immunogenic carrier.

9. The conjugate of claim 8 which is obtainable by coupling the compound of any one of claims 1 to 3 via group X to the immunogenic carrier.

10. The conjugate of claim 8 or 9, wherein the immunogenic carrier is a protein, a peptide, an oligonucleotide or a polymer.

11. The conjugate of claim 10, wherein the protein is ovalbumin, tetanus toxoid, diphtheria toxoid, keyhole limpet hemocyanin or CRM₁₉₇.

12. A method for the preparation of the conjugate of any one of claims 8 to 11, comprising:
a) activating the immunogenic carrier; and
b) coupling the activated immunogenic carrier obtained in step a) to the compound of any one of claims 1 to 3.

13. The method of claim 12 wherein in step a) the immunogenic carrier is activated as a succinimid ester and group X of the compound of any of claims 1 to 3 is NH₂.

14. A method for the preparation of the conjugate of any of claims 8 to 11, comprising:
a) activating group X of the compound of any of claims 1 to 3; and
b) coupling the activated compound obtained in step a) to the immunogenic carrier.

15. The method of claim 14 wherein in step a) group X of the compound of any of claims 1 to 3 is activated as a maleimide group.

16. An antibody specifically binding to the compound of any one of claims 1 to 3, NNK and non-detoxified metabolites thereof not having an N-oxidized pyridyl group, wherein the specific binding of said antibody to NNK and non-detoxified metabolites thereof is defined as an at least 100 times higher affinity to NNK and non-detoxified metabolites thereof than to detoxified derivatives of NNK having an N-oxidized pyridyl group, wherein said antibody binds to the compound of any one of claims 1 to 3 bound to an immunogenic carrier with a dissociation constant K_{D} of less than 100 nM.

17. The antibody of claim 16, which does not bind to derivatives of NNK and/or NNAL having an N-oxidized pyridyl group.

18. A method for producing the antibody of claim 16 or 17, comprising immunizing a non-human animal with the conjugate of any one of claims 8 to 11.

19. An antibody wherein CDR1 of V_{H} has the sequence of [SEQ ID NO: 3], CDR2 of V_{H} has the sequence of [SEQ ID NO: 4], CDR3 of V_{H} has the sequence of [SEQ ID NO: 5], CDR1 of V_{L} has the sequence of [SEQ ID NO: 6], CDR2 of V_{L} has the sequence of [SEQ ID NO: 7] and CDR3 of V_{L} has the sequence of [SEQ ID NO: 8].

20. An antibody wherein CDR1 of V_{H} has the sequence of [SEQ ID NO: 11], CDR2 of V_{H} has the sequence of [SEQ ID NO: 12], CDR3 of V_{H} has the sequence of [SEQ ID NO: 13], CDR1 of V_{L} has the sequence of [SEQ ID NO: 14], CDR2 of V_{L} has the sequence of [SEQ ID NO: 15] and CDR3 of V_{L} has the sequence of [SEQ ID NO: 16].

21. A pharmaceutical composition comprising the conjugate of any one of claims 8 to 11.

22. The pharmaceutical composition of claim 18, which is a vaccine.

23. The conjugate of any one of claims 8 to 11 for treating and/or preventing disorders associated with tobacco specific nitrosamine exposure.

24. The conjugate of claim 23, wherein the disorder is a cancerous disease.

25. A method of detecting NNK, a non-detoxified metabolite thereof not having an N-oxidized pyridyl group or an adduct comprising a pyridyloxobutyl group derived from NNK, comprising subjecting a sample to the antibody of claim 16 or 17 and detecting binding of the antibody to NNK or a non-detoxified metabolite thereof.

26. The method of claim 25, wherein the sample is obtained from a subject.

27. A method for the detection of antibodies specific for NNK or a non-detoxified metabolite thereof, comprising subjecting a sample from a subject to the conjugate of any one of claims 8 to 11 and detecting binding of the conjugate to the antibody.


SEQUENCE LISTING

<110> Centre de Recherche Publique de la Santé (CRP-Santé)
Muller, Claude P.

<120> Conjugates for the preparation of antibodies specific for
4-(methylnitrosamino)-1-(3-pyridyl)-1-butanone and non-detoxified
metabolites thereof

<130> N1640 EP

<160> 16

<170> PatentIn version 3.3

<210> 1
<211> 119
<212> PRT
<213> artificial sequence

<220>
<221> source
<223> /note="Description of artificial sequence:VH of antibody produced in
mouse"

<400> 1

Glu Val Gln Val Val Glu Ser Gly Gly Gly Phe Val Arg Pro Gly Gly
1               5                   10                  15

Ser Leu Lys Leu Ser Cys Ala Ala Ser Gly Phe Thr Phe Ser Asn Tyr
            20                  25                  30

Val Met Ser Trp Leu Arg Gln Thr Pro Glu Lys Arg Leu Glu Trp Val
        35                  40                  45

Ala Ser Ile Ser Ser Gly Gly Ser Thr Tyr Tyr Pro Asp Ser Met Lys
    50                  55                  60

Gly Arg Phe Thr Ile Ser Arg Asp Asn Ala Arg Asn Ile Leu Tyr Leu
65                  70                  75                  80

Gln Met Ser Ser Leu Arg Ser Glu Asp Thr Ala Met Phe Tyr Cys Ala
                85                  90                  95

Arg Trp Asp Tyr Arg Tyr Val Tyr Tyr Pro Leu Asp Tyr Trp Gly Gln
            100                 105                 110

Gly Thr Ser Val Thr Val Ser
        115

<210> 2
<211> 111
<212> PRT
<213> artificial sequence

<220>
<221> source
<223> /note="Description of artificial sequence:VL of antibody produced in
mouse"

<400>  2

Asp Ile Val Leu Ser Gln Ser Pro Ala Leu Val Ser Ala Ser Pro Gly
1               5                   10                  15

Glu Arg Val Thr Met Thr Cys Ser Ala Ser Ser Ser Val Asn Tyr Ile
            20                  25                  30

Tyr Trp Tyr Gln Gln Lys Pro Arg Ser Ser Pro Lys Pro Trp Ile Tyr
        35                  40                  45

Leu Thr Ser Asn Leu Ala Ser Gly Val Pro Val Arg Phe Ser Gly Ser
    50                  55                  60

Gly Ser Gly Thr Ser Tyr Ser Leu Thr Ile Thr Ser Met Glu Ala Glu
65                  70                  75                  80

Asp Ala Ala Thr Tyr Tyr Cys Gln Gln Trp Thr Ser Asn Pro Pro Ala
                85                  90                  95

Leu Thr Phe Gly Ala Gly Thr Lys Leu Glu Leu Lys Arg Arg Pro
            100                 105                 110

<210>  3
<211>  10
<212>  PRT
<213>  artificial sequence

<220>
<221>  source
<223>  /note="Description of artificial sequence:CDR1 of VH of antibody produced
in mouse"

<400>  3

Gly Phe Thr Phe Ser Asn Tyr Val Met Ser
1               5                   10

<210>  4
<211>  9
<212>  PRT
<213>  artificial sequence

<220>
<221>  source
<223>  /note="Description of artificial sequence:CDR2 of VH of antibody produced
in mouse"

<400>  4

Ser Ile Ser Ser Gly Gly Ser Thr Tyr
1               5

<210>  5
<211>  12
<212>  PRT
<213>  artificial sequence

<220>
<221>  source
<223>  /note="Description of artificial sequence:CDR3 of VH of antibody produced
in mouse"

<400>  5

Trp Asp Tyr Arg Tyr Val Tyr Tyr Pro Leu Asp Tyr
1               5                   10


<210>  6
<211>  10
<212>  PRT
<213>  artificial sequence

<220>
<221>  source
<223>  /note="Description of artificial sequence:CDR1 of VL of antibody produced
in mouse"

<400>  6

Ser Ala Ser Ser Ser Val Asn Tyr Ile Tyr
1               5                   10


<210>  7
<211>  7
<212>  PRT
<213>  artificial sequence

<220>
<221>  source
<223>  /note="Description of artificial sequence:CDR2 of VL of antibody produced
in mouse"

<400>  7

Leu Thr Ser Asn Leu Ala Ser
1               5


<210>  8
<211>  11
<212>  PRT
<213>  artificial sequence

<220>
<221>  source
<223>  /note="Description of artificial sequence:CDR3 of VL of antibody produced
in mouse"

<400>  8

Gln Gln Trp Thr Ser Asn Pro Pro Ala Leu Thr
1               5                   10


<210>  9
<211>  119
<212>  PRT
<213>  artificial sequence

<220>
<221>  source
<223>  /note="Description of artificial sequence:VH of antibody produced in
mouse"

<400>  9

Glu Val Gln Leu Val Glu Ser Gly Gly Gly Phe Val Lys Pro Gly Gly
1               5                   10                  15

Ser Leu Lys Leu Ser Cys Ala Ala Ser Gly Phe Thr Phe Ser Asn Phe
            20                  25                  30

Val Met Ser Trp Val Arg Gln Thr Pro Glu Lys Arg Leu Gln Trp Val
        35                  40                  45

Ala Ser Ile Ser Ser Gly Gly Ser Thr Tyr Tyr Pro Asp Ile Leu Arg
    50                  55                  60

Gly Arg Phe Thr Ile Ser Arg Asp Asn Gly Arg Asn Ile Leu Tyr Leu
65                  70                  75                  80

Gln Met Ser Ser Leu Arg Ser Glu Asp Thr Ala Met Tyr Tyr Cys Ser
                85                  90                  95

Arg Trp Asp Tyr Arg Tyr Val Tyr Tyr Pro Leu Asp Tyr Trp Gly Gln
            100                 105                 110

Gly Thr Ser Val Thr Val Ser
        115

<210>  10
<211>  111
<212>  PRT
<213>  artificial sequence

<220>
<221>  source
<223>  /note="Description of artificial sequence:VL of antibody produced in
mouse"

<400>  10

Asp Ile Val Leu Thr Gln Ser Pro Val Leu Met Ser Ala Ser Pro Gly
1               5                   10                  15

Glu Lys Val Thr Met Thr Cys Ser Ala Ser Ser Ser Val Ser Tyr Ile
            20                  25                  30

Tyr Trp Tyr Gln Gln Lys Pro Gly Ser Pro Pro Lys Pro Trp Ile Tyr
        35                  40                  45

Leu Thr Ser Thr Leu Ala Ser Gly Val Pro Val Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Arg Ser His Pro Pro Ala

85                  90                  95

Leu Thr Phe Gly Ala Gly Thr Lys Leu Glu Ile Lys Arg Arg Pro
            100                 105                 110

<210>  11
<211>  10
<212>  PRT
<213>  artificial sequence

<220>
<221>  source
<223>  /note="Description of artificial sequence:CDR1 of VH of antibody produced
in mouse"

<400>  11

Gly Phe Thr Phe Ser Asn Phe Val Met Ser
1               5                   10

<210>  12
<211>  9
<212>  PRT
<213>  artificial sequence

<220>
<221>  source
<223>  /note="Description of artificial sequence:CDR2 of VH of antibody produced
in mouse"

<400>  12

Ser Ile Ser Ser Gly Gly Ser Thr Tyr
1               5

<210>  13
<211>  12
<212>  PRT
<213>  artificial sequence

<220>
<221>  source
<223>  /note="Description of artificial sequence:CDR3 of VH of antibody produced
in mouse"

<400>  13

Trp Asp Tyr Arg Tyr Val Tyr Tyr Pro Leu Asp Tyr
1               5                   10

<210>  14
<211>  10
<212>  PRT
<213>  artificial sequence

<220>
<221>  source
<223>  /note="Description of artificial sequence:CDR1 of VL of antibody produced
in mouse"

<400>  14

Ser Ala Ser Ser Ser Val Ser Tyr Ile Tyr

1               5                   10


<210>  15
<211>  7
<212>  PRT
<213>  artificial sequence

<220>
<221>  source
<223>  /note="Description of artificial sequence:CDR 2 of VL of antibody
produced in mouse"

<400>  15

Leu Thr Ser Thr Leu Ala Ser
1               5


<210>  16
<211>  11
<212>  PRT
<213>  artificial sequence

<220>
<221>  source
<223>  /note="Description of artificial sequence:CDR3 of VL of antibody produced
in mouse"

<400>  16

Gln Gln Trp Arg Ser His Pro Pro Ala Leu Thr
1               5                   10